# EUROPEAN PATENT APPLICATION

(11) **EP 2 599 767 A1**
(43) Date of publication of application: **05.06.2013**
(21) Application number: 11010059.1
(22) Date of filing: 21.12.2011
(51) Int. Cl.: C07C 69/14, C07C 69/612, C07C 69/86, C07C 229/36, C07C 233/51, A61K 31/216

(54) **Phenylbutyl-derivatives**

(30) Priority: 30.11.2011 EP 11009472
(71) Applicant: Lunamed AG, 7000 Chur (CH)
(72) Inventor: Truog, Peter, 7000 Chur (CH); Buschmann, Helmut H., 52076 Aachen (Walheim) (DE)
(74) Representative: Peters, Hajo

(57) **Abstract**

The present invention refers to derivatives of formula (I), wherein R⁴, R⁵, Y, and n are defined in claim 1, their stereoisomers as well as the acids, bases or salts thereof, medicaments comprising them, as well as said compounds for use in cancer therapy and other phamaceutical applications.

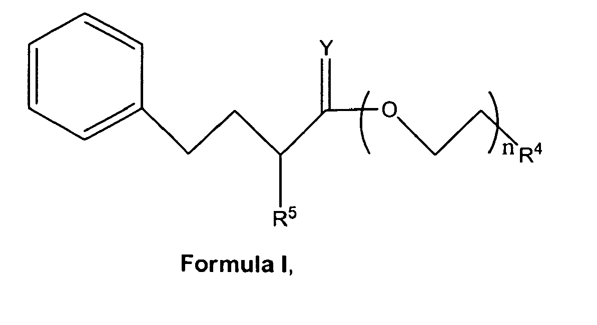

## Description

### Field of the invention

The present invention refers to Derivatives of Phenylbutyl, stereoisomers as well as the acids, bases or salts thereof, medicaments comprising them, as well as their use in cancer therapy and other pharmaceutical applications.

### Background of the invention

One phenylbutyl-derivative, phenylbutyric acid is a well known compound and is marketed in form of its sodium salt as a drug in the USA and European Union. Sodium phenylbutyrate is an orphan drug, marketed by Ucyclyd Pharma (Hunt Valley, USA) under the trade name Buphenyl and by Swedish Orphan International (Sweden) as Ammonaps for the treatment of urea cycle disorders.

In addition, 4-phenylbutyric acid sodium salt (sodium 4-phenylbutyrate) has also been described in patents ans scientific literature in a number of medical uses. These use encompass the treatment of a variety of illnesses, such as benign prostate hyperplasy, cancer, cystic fibrosis, HIV, kidney and liver failures, and thalasemia. For example, WO 93/0786, WO 9510271 or EP 725635 (Samid) disclose compositions and methods using phenylacetic acid derivatives for therapy and prevention of a number of pathologies, including cancer, AIDS, anemia, and severe beta-chain hemoglobinopathies.

One of the major drawbacks of 4-Phenylbutyric acid is that it is relatively fast metabolized in the human body to phenylacetic acid. This fact influences and challenges treatment schedules and thus leads to problems in applying a proper treatment with 4-Phenylbutyric acid and its salts.

Accordingly, it would be desirable to have a phenylbutyl derivative like a derivative of 4-phenylbutyrate that avoids the problems associated with application of 4-phenylbutyric acid or its salts. It would be even more preferable to find a phenylbutyl/4-phenylbutyrate derivative whose activity is enhanced by an additional factor resting in the molecular structure of the derivative. Thus, it was the goal of this invention to identify alternative or improved derivatives of phenylbutyl or phenylbutyric acid. Thus, it was the goal of this invention to identify alternative or improved derivatives of phenylbutyl or phenylbutyric acid.

It has now been found that certain selected derivatives of phenylbutyl like ester derivatives of phenylbutyric acid as described below would solve this problem showing very advantageous attributes.

Accordingly, the invention is drawn to one or more phenylbutyl derivatives (ester derivatives of phenylbutyric acid) of Formula I 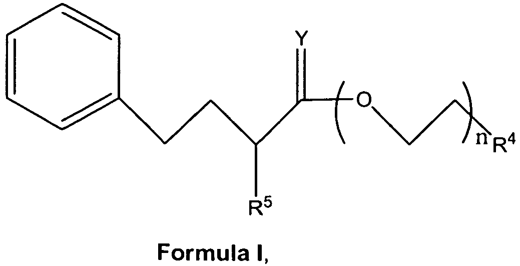 wherein
n is selected from 0, or from 1, 2, 3, 4, 5, 6, 7, 8, 9 and 10;
Y is selected from O or H₂;
R⁵ is selected from H or NH₂; and
R⁴ is selected from either a salicylic acid derivative, radical of an amino acid or 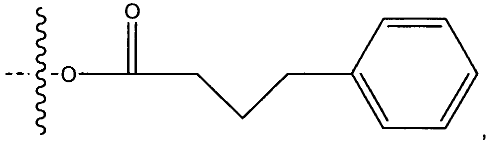 with R⁴ being optionally connected to the core by one or more amino acids;
optionally in form of one of its stereoisomers, enantiomers or diastereomers, its racemate or in form of a mixture of at least two of its stereoisomers in any mixing ratio;
in form of the free acid, free base or salt.

"Amino acid" according to the invention is defined as any of the naturally occurring amino acids as well as any synthetic amino acid, preferably is defined as naturally occurring amino acids. Accordingly "radical of an amino acid" is a radical/substituent derived from an amino acid as defined above.

In a preferred embodiment of the phenylbutyl-derivative of Formula I according to the invention
R⁴ is selected from any of the following structures, which may optionally be substituted by one or more of F, Cl, Br, I, NH₂, OH, SH, CH₃, C₂H₅, C₃H₇, C₄H₉, CH₂Cl, CHCl₂, CF₃, CHF₂, CH₂F: 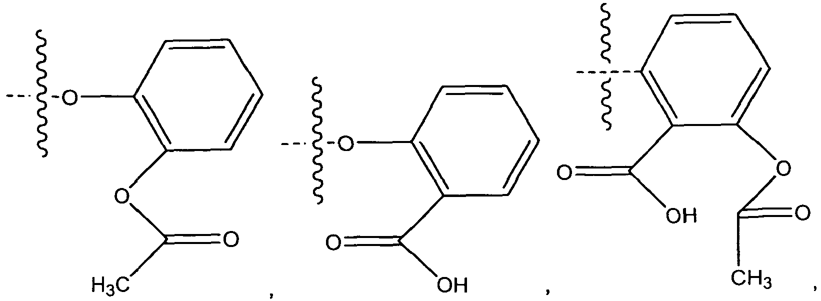 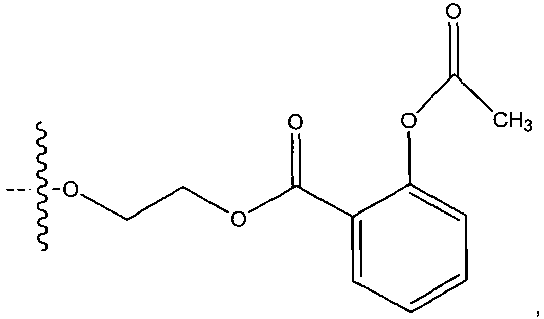 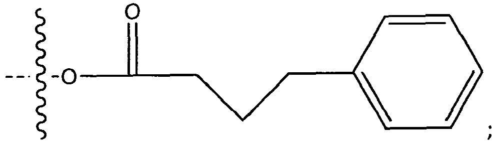 a radical of an amino acid, or
with n # 0 and Y being 0 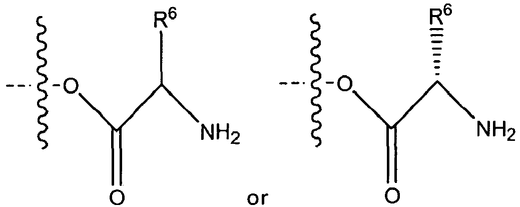 with n being 0 and Y being O

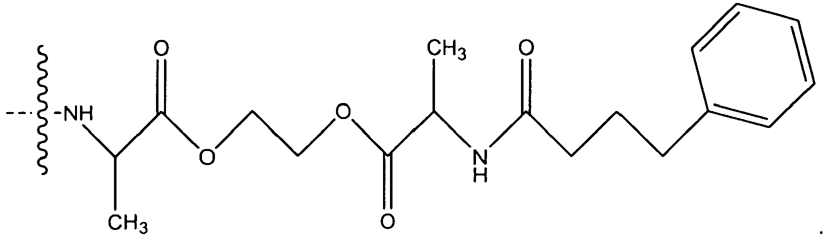

In another preferred embodiment of the phenylbutyl-derivative of Formula I according to the invention
R⁴ is selected from any of the following structures 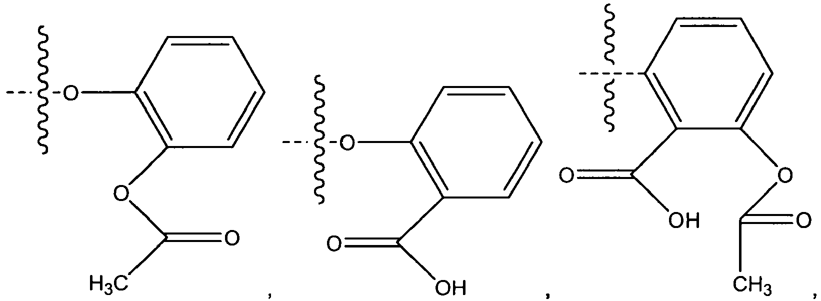 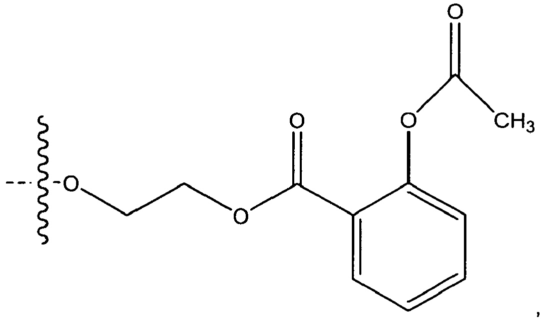 or
a radical of an amino acid, or
with n ≠ 0 and Y being O 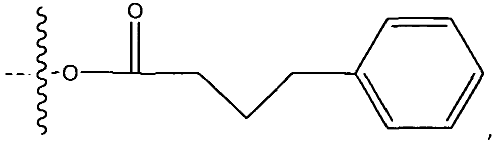 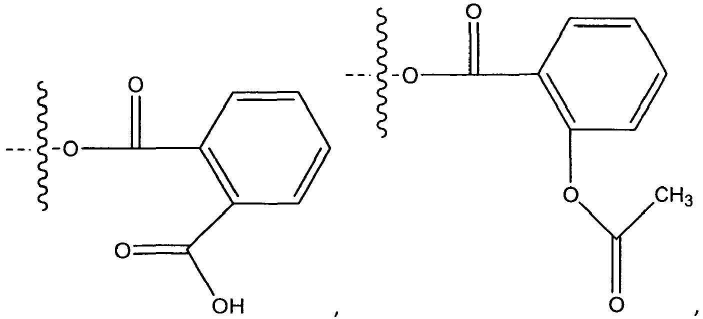 or 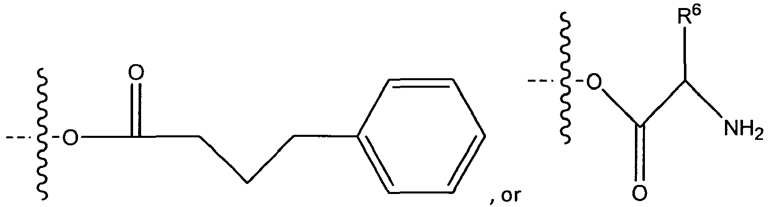 with n being 0 and Y being O 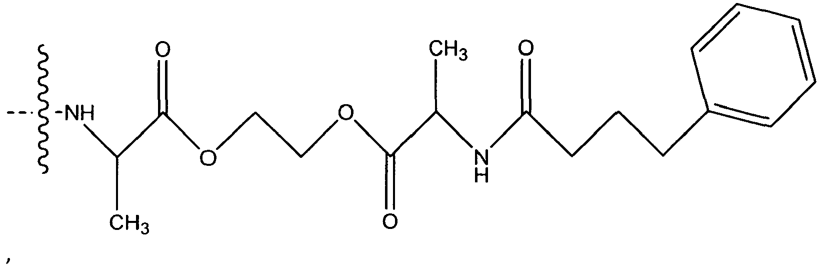 or 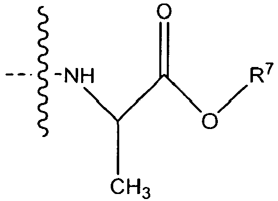 R⁶ being selected from H 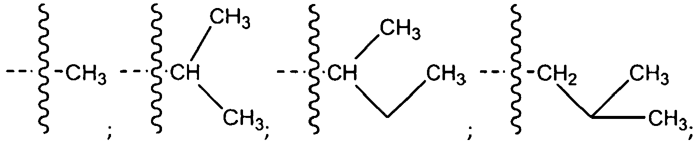 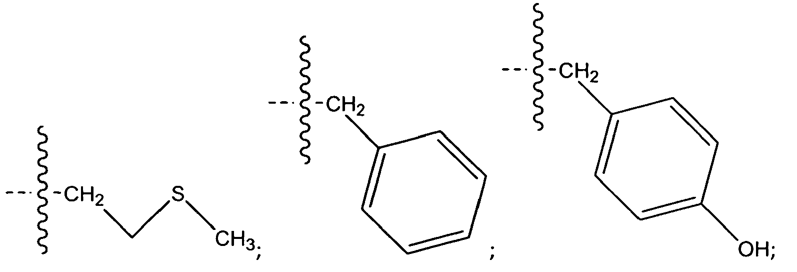 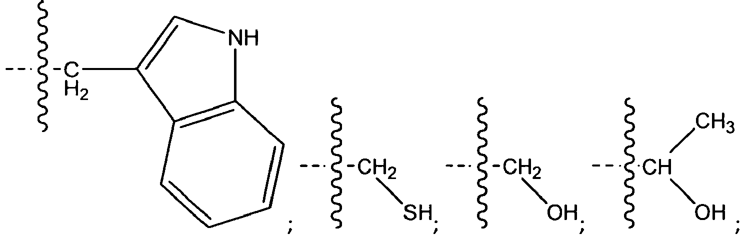 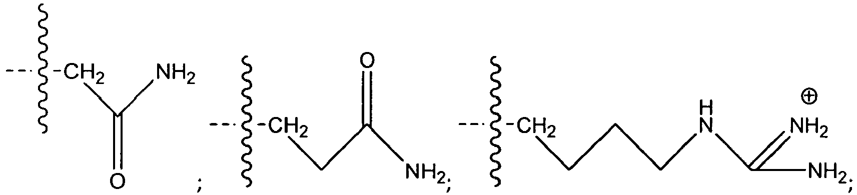 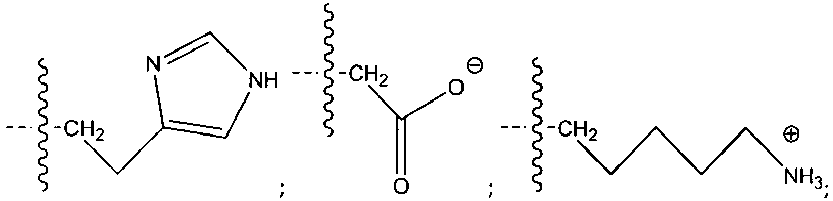 or 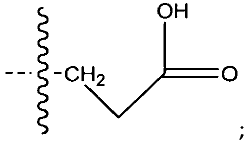 R⁷ being selected from H, C₁₋₄-Alkyl; or 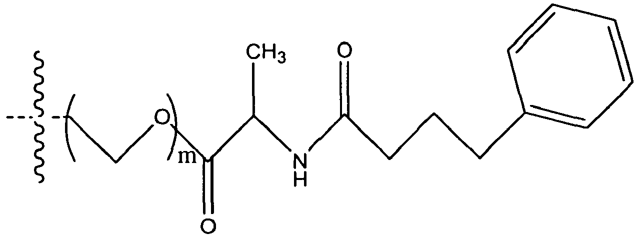 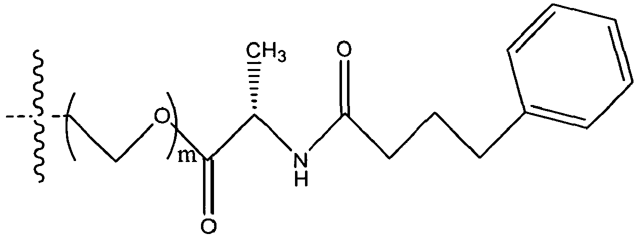 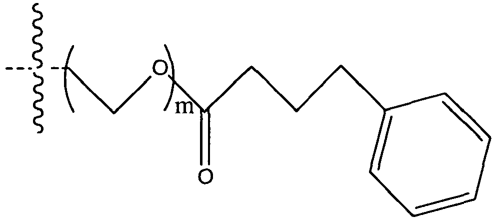 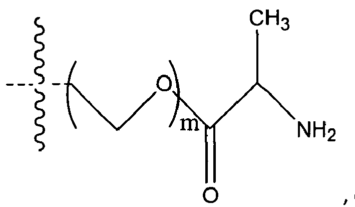 or

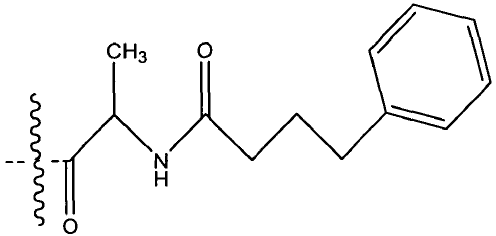

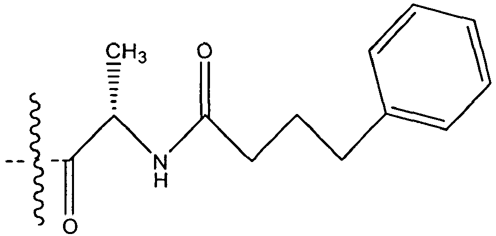

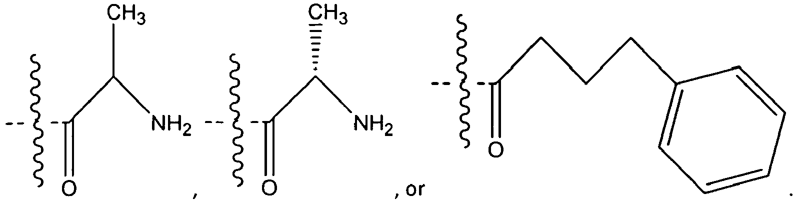

In a further preferred embodiment of the phenylbutyl-derivative of Formula I according to the invention
R⁴ is selected from any of the following structures 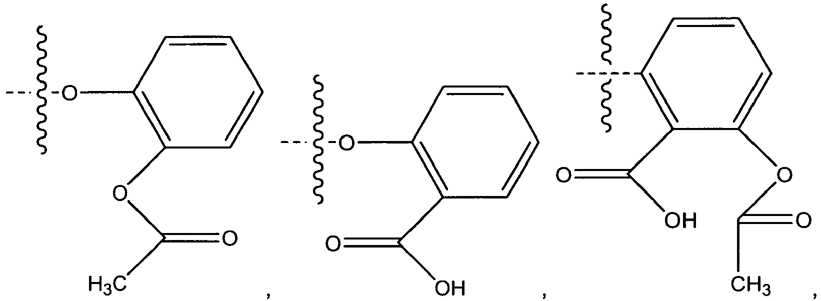 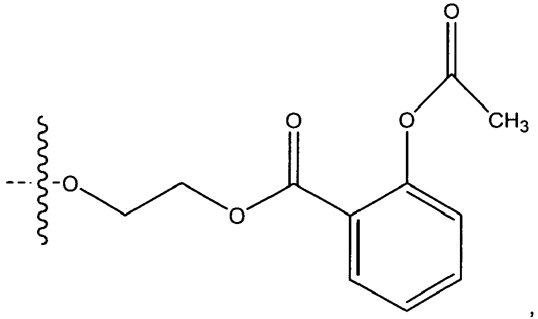 or
a radical of a D-amino acid, or
with n ≠ 0 and Y being O 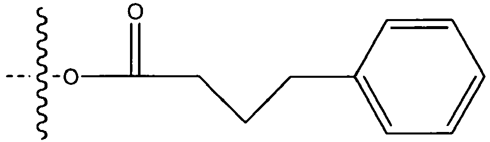 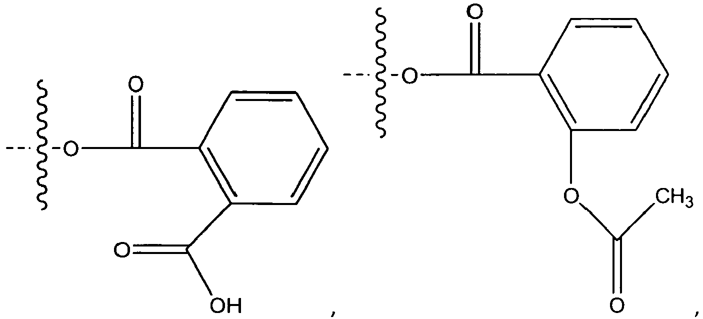 or 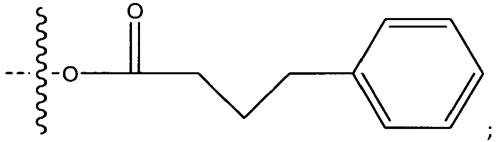 or 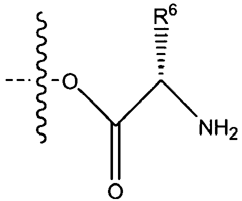 (being a D-configuration)
with n being 0 and Y being O 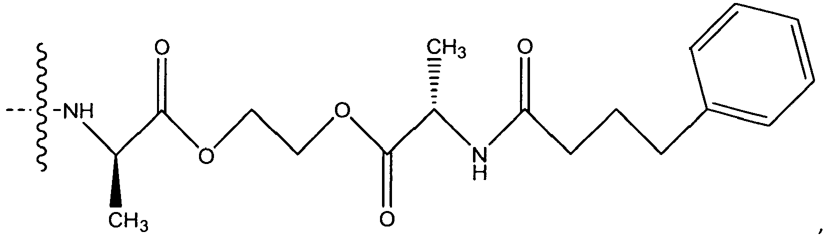 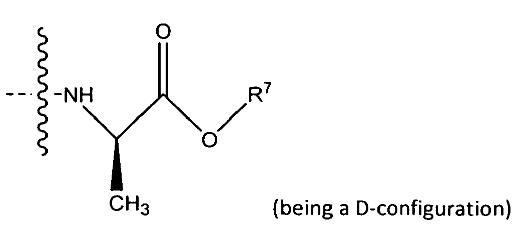 R⁶ being selected from H 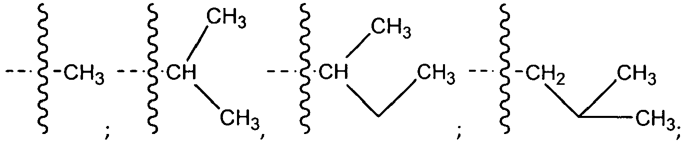 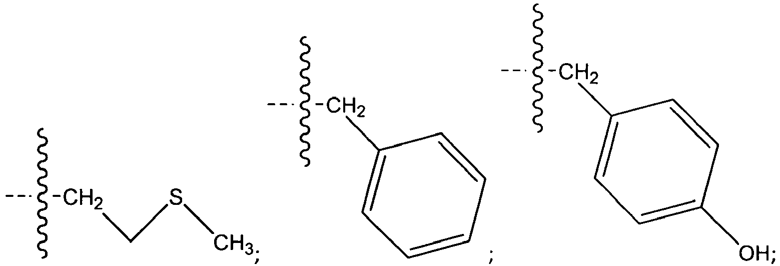 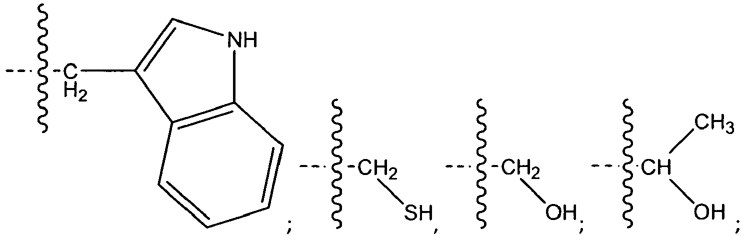 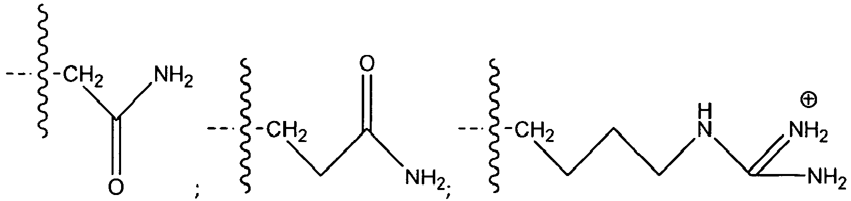 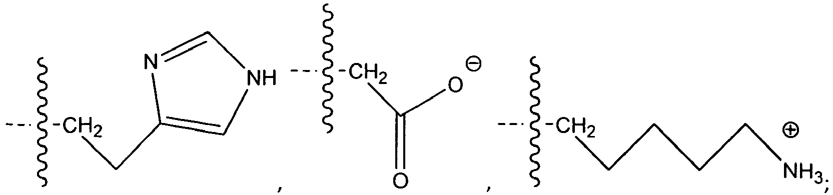 or 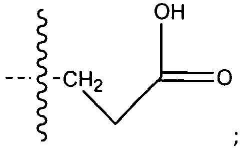 R⁷ being selected from H, C₁₋₄-Alkyl; or

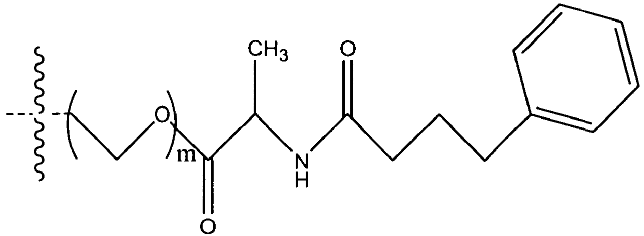

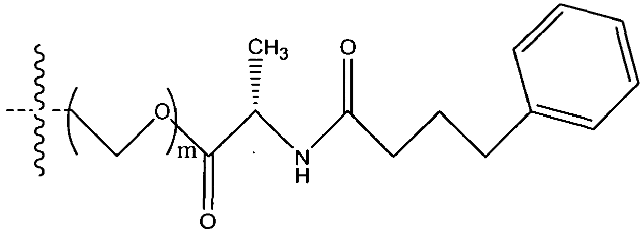

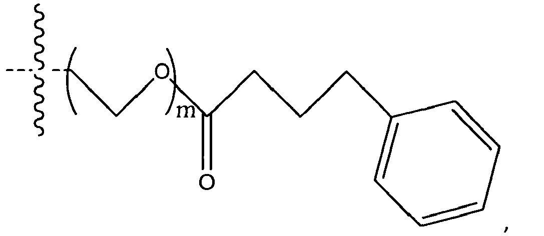

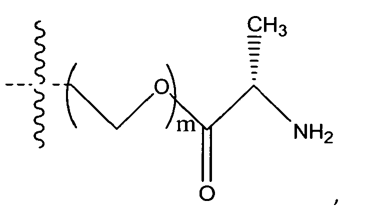

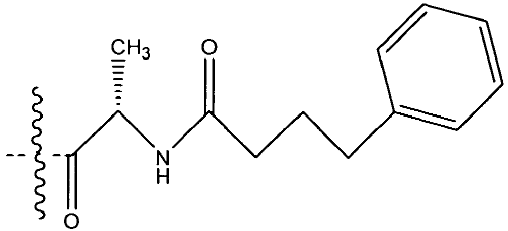

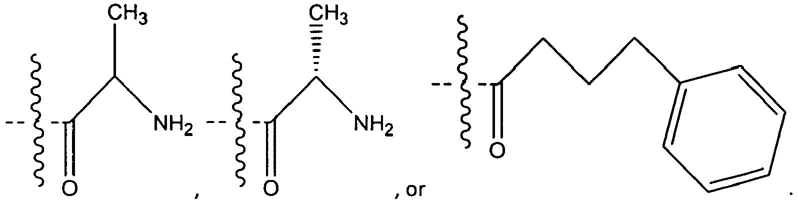

In a further preferred embodiment of the phenylbutyl-derivative of Formula I according to the invention Y is O.

In a further preferred embodiment of the phenylbutyl-derivative of Formula I according to the invention R⁵ is H.

In a further preferred embodiment of the phenylbutyl-derivative of Formula I according to the invention, when Y is O and n ≠ 0:
R⁴ is selected from any of the following structures 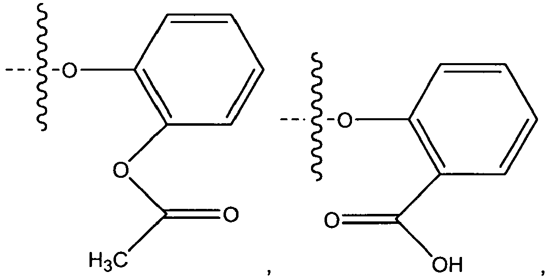 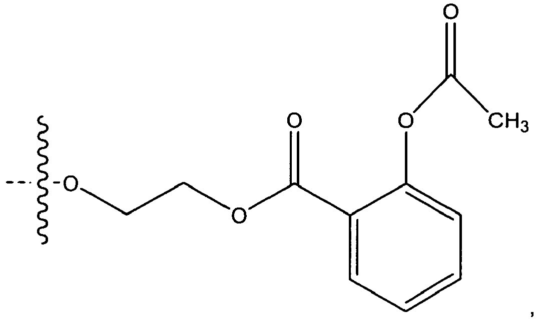 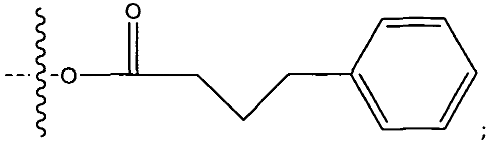 or 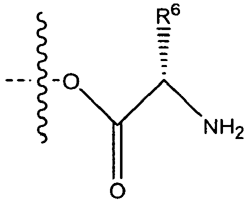 (being a D-configuration),
R⁶ being selected from H 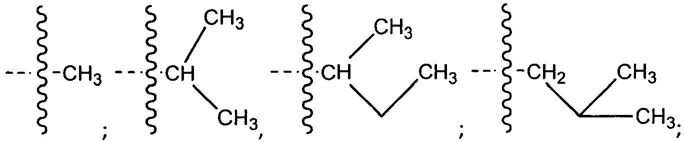 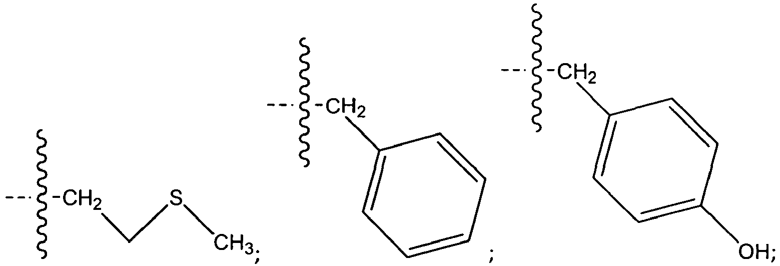 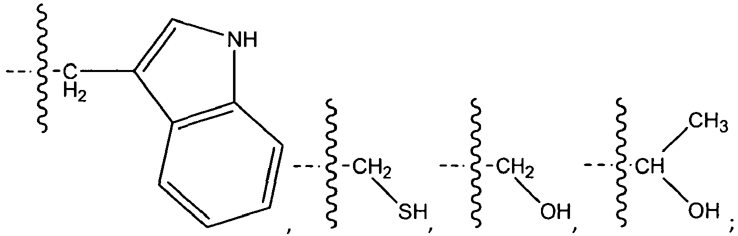 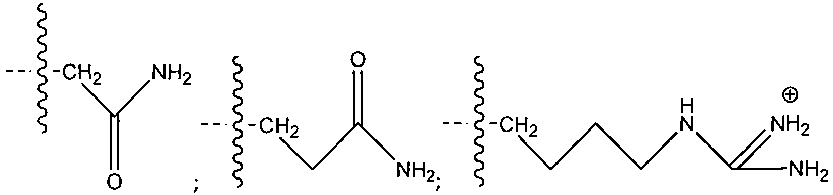 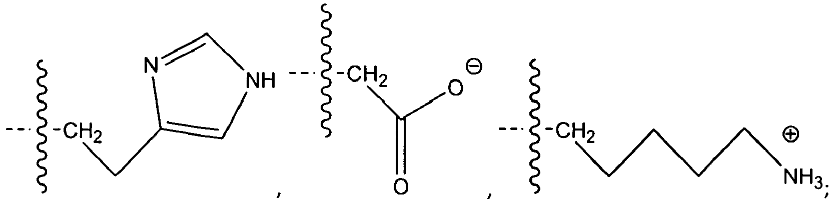 or

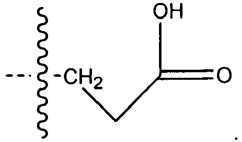

In a further preferred embodiment of the phenylbutyl-derivative of Formula I according to the invention n is 1, 2, 3, 4, 5, or 6, preferably is 1, 2, 3, or 4, or is 1 or 2.

In a further preferred embodiment of the phenylbutyl-derivative of Formula I according to the invention, when Y is O and n is 0:
R⁴ is selected from any of the following structures 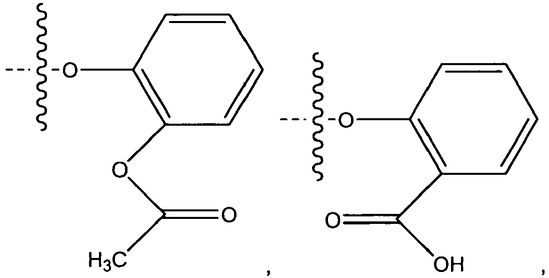 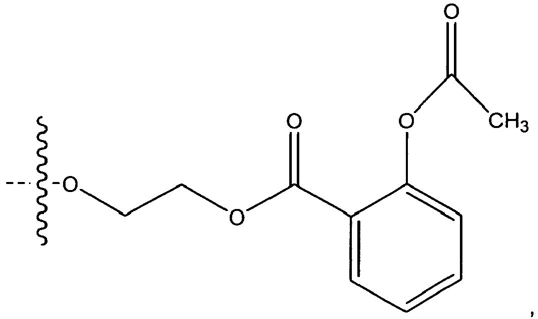 or 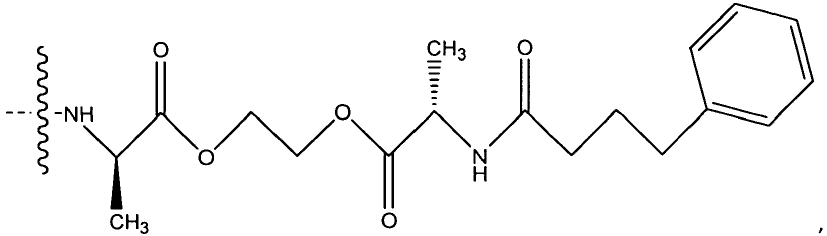 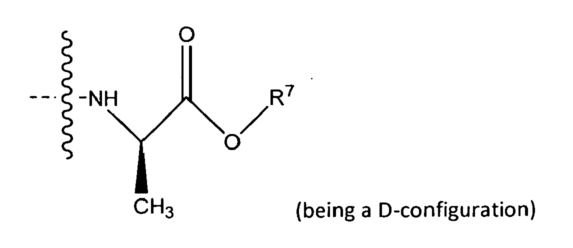
R⁶ being selected from H 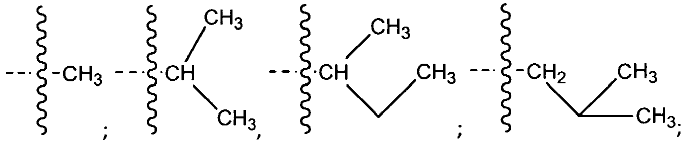 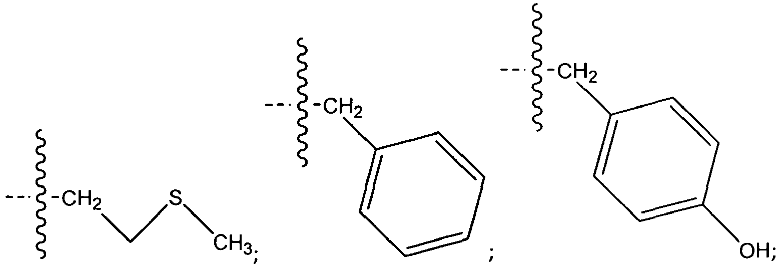 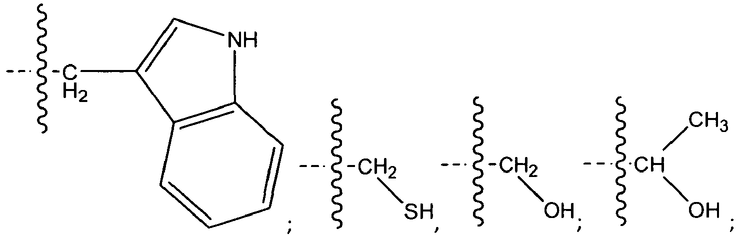 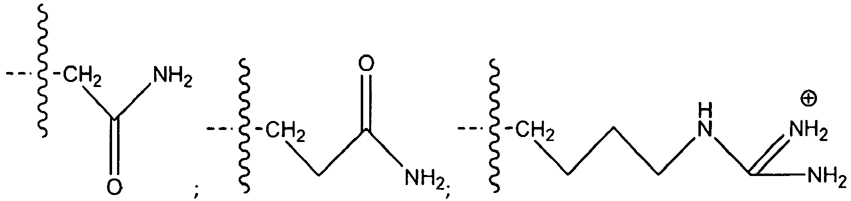 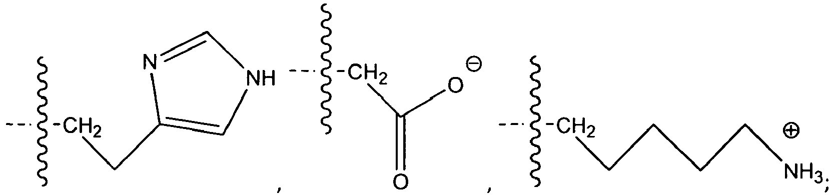 or 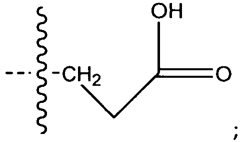
R⁷ being selected from H, C₁₋₄-Alkyl; or 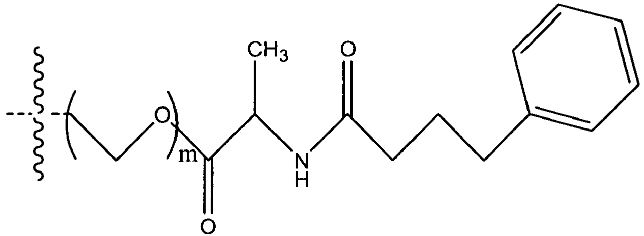 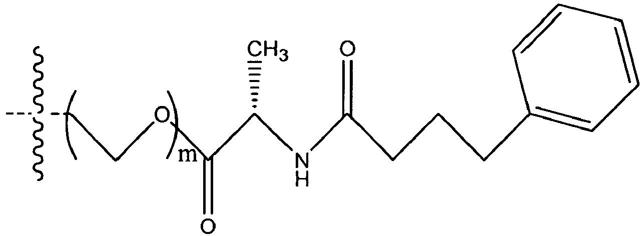 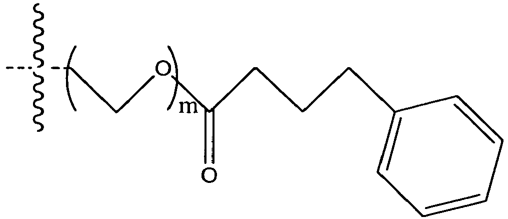 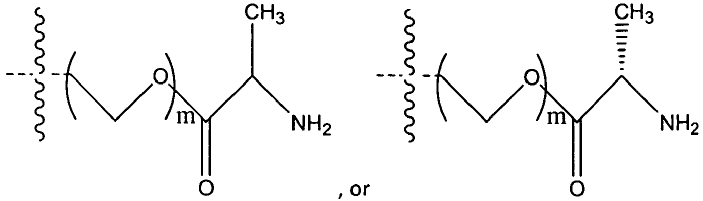 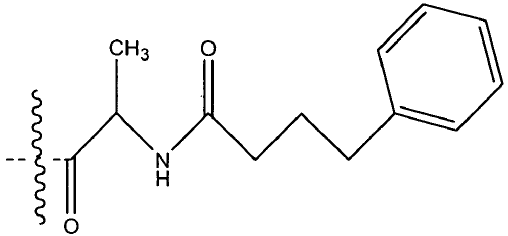 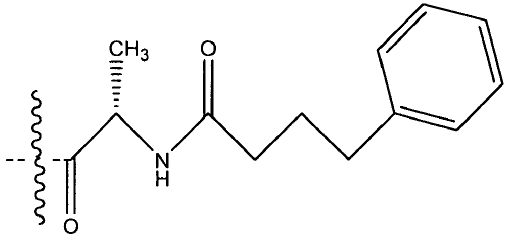 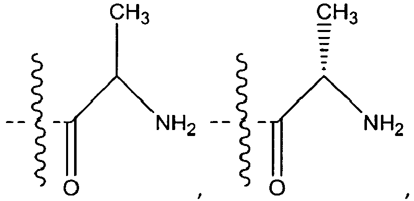 or

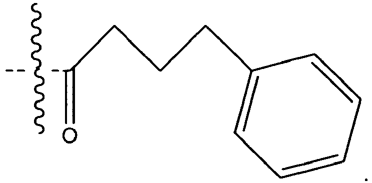

In a further preferred embodiment the phenylbutyl-derivative of Formula I according to the invention is a compound of Formula II 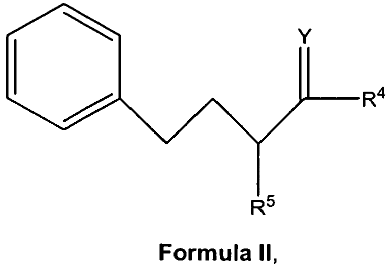 wherein
Y is selected from O or H₂;
R⁵ is selected from H or NH₂; and
R⁴ is selected from a derivative of either a salicylic acid, phenyl butyric acid, ester or ether or a radical of a D-amino acid, being optionally connected to the core by polyethyleneglycol, one or more amino acids or mixtures thereof;
optionally in form of one of its stereoisomers, enantiomers or diastereomers, its racemate or in form of a mixture of at least two of its stereoisomers in any mixing ratio;
in form of the free acid, free base or salt.

In a preferred embodiment of the phenylbutyl-derivative of Formula II according to the invention
R⁴ is selected from any of the following structures, which may optionally be substituted by one or more of F, Cl, Br, I, NH₂, OH, SH, CH₃, C₂H₅, C₃H₇, C₄H₉, CH₂Cl, CHCl₂, CF₃, CHF₂, CH₂F: 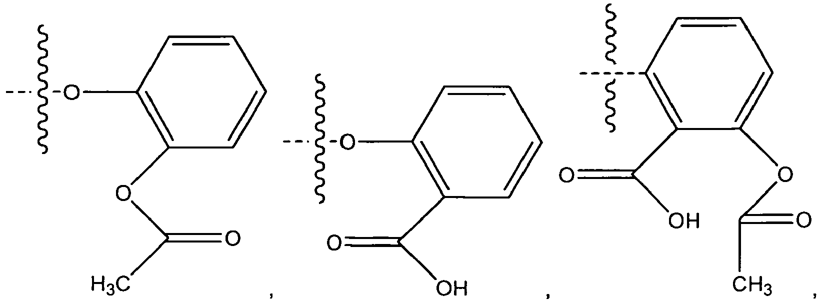 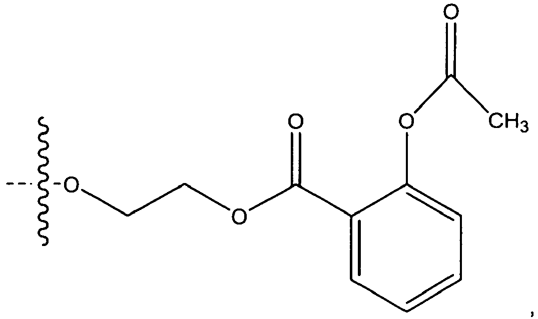 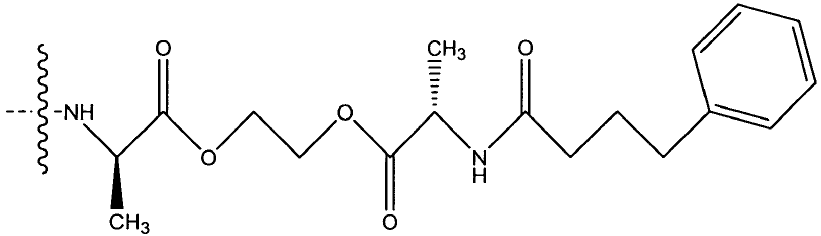 , a radical of a D-amino acid, or

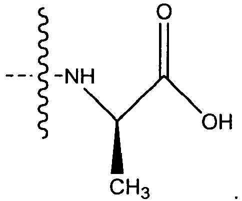

In a further preferred embodiment of the phenylbutyl-derivative of Formula I or Formula II according to the invention
R⁴ is selected from any one of the following structures: 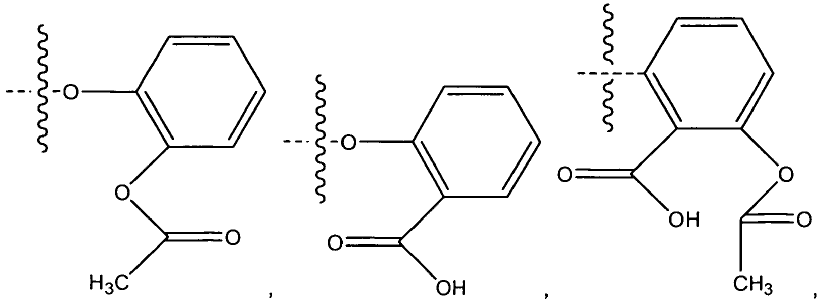 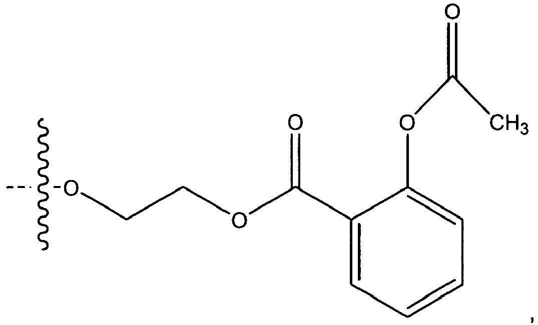 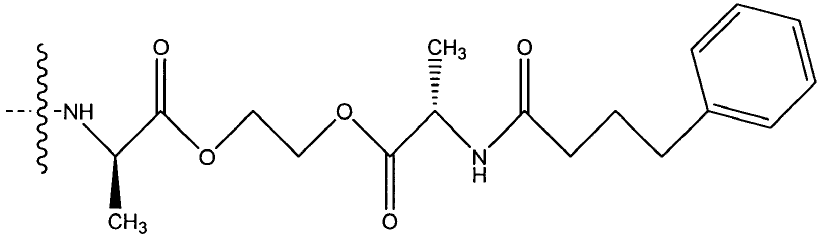 , or

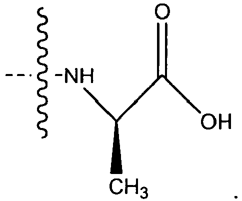

In a further preferred embodiment the phenylbutyl-derivative of Formula I or II according to the invention is selected from 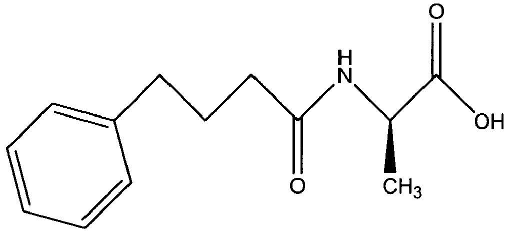 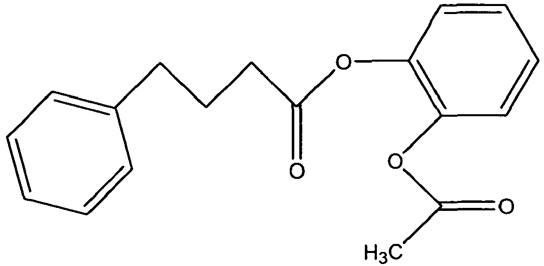 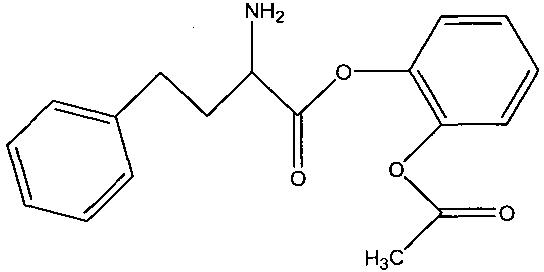 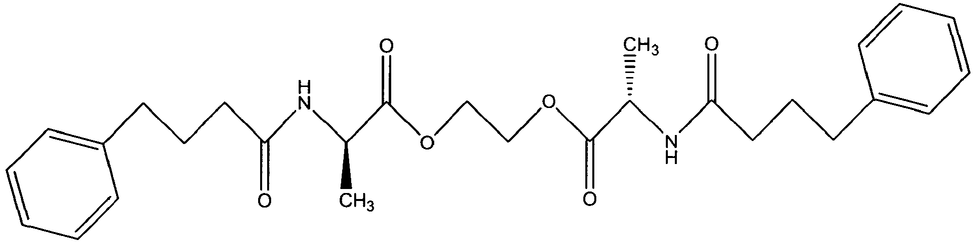 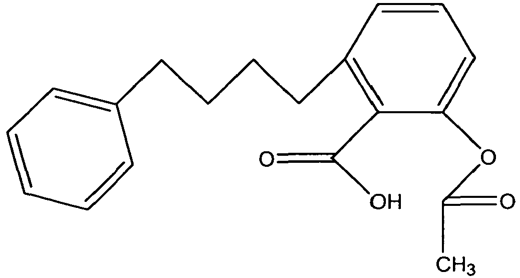 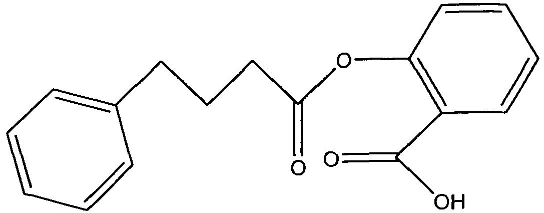 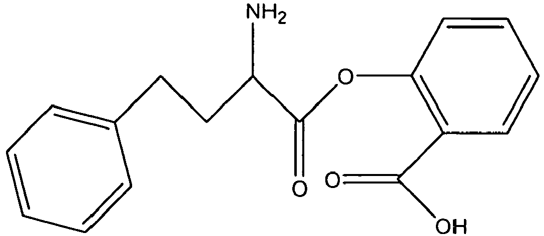 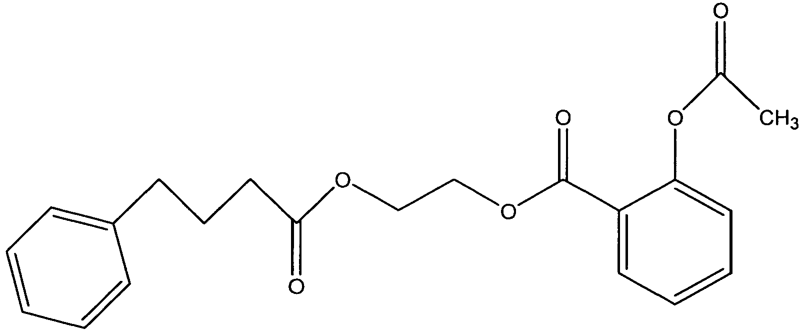 , wherein the compound may optionally be substituted by one or more of F, Cl, Br, I, NH₂, OH, SH, CH₃, C₂H₅, C₃H₇, C₄H₉, CH₂Cl, CHCl₂, CF₃, CHF₂, CH₂F, preferably is unsubstituted;
optionally in form of one of its stereoisomers, enantiomers or diastereomers, its racemate or in form of a mixture of at least two of its stereoisomers in any mixing ratio;
in form of the free acid, free base or salt.

Phenylbutyl-derivatives of Formula I or II according to the invention are physiologically well-tolerated. Accordingly, a further aspect of the invention refers to a Pharmaceutical formulation comprising at least one Phenylbutyl-Derivative of Formula I (or of Formula II) according to the invention and optionally at least one physiologically acceptable excipient.

A further aspect of the invention refers to a Phenylbutyl-Derivative of Formula I (or of Formula II) according to the invention for use in the treatment of cancer, mucositis, amyotropic lateral sclerosis (ALS), skin/mucosal itching, degenerative diseases of the eye, neurodegenerative disorders, Huntington's disease, eating disorders, obesity, drug induced weight gain, pruritis, alcoholism, promyelocytic leukemia, especially acute promyelocytic leukemia, cardiac injury, especially adriamycin induced cardiac injury, epithelial damage, connective tissue damage, desmosis, Parkinson Disease, myelodysplastic syndrome, urea cycle disorder, fibrotic lung diseases, hepatic encephalopathies, thioredoxin mediated diseases, human papilloma virus (HPV) infections, autoimmune diseases, thalassemia, genetic disorders; or in the regulation of HDAC6 activity, the modulation of stem cells, the prevention of grey hair, the promotion of neuronal growth, the prevention of bone and joint diseases, or as adjuvant in cancer therapy.

A parallel further aspect of the invention refers to the use of a Phenylbutyl-Derivative of Formula I (or of Formula II) according to the invention in the manufacture of a medicament for the treatment of cancer, mucositis, amyotropic lateral sclerosis (ALS), skin/mucosal itching, degenerative diseases of the eye, neurodegenerative disorders, Huntington's disease, eating disorders, obesity, drug induced weight gain, pruritis, alcoholism, promyelocytic leukemia, especially acute promyelocytic leukemia, cardiac injury, especially adriamycin induced cardiac injury, epithelial damage, connective tissue damage, desmosis, Parkinson Disease, myelodysplastic syndrome, urea cycle disorder, fibrotic lung diseases, hepatic encephalopathies, thioredoxin mediated diseases, human papilloma virus (HPV) infections, autoimmune diseases, thalassemia, genetic disorders; or for the regulation of HDAC6 activity, the modulation of stem cells, the prevention of grey hair, the promotion of neuronal growth, the prevention of bone and joint diseases, or for use as adjuvant in cancer therapy.

A parallel further aspect of the invention refers to a method of treating a being (human or animal) suffering from cancer, mucositis, amyotropic lateral sclerosis (ALS), skin/mucosal itching, degenerative diseases of the eye, neurodegenerative disorders, Huntington's disease, eating disorders, obesity, drug induced weight gain, pruritis, alcoholism, promyelocytic leukemia, especially acute promyelocytic leukemia, cardiac injury, especially adriamycin induced cardiac injury, epithelial damage, connective tissue damage, desmosis, Parkinson Disease, myelodysplastic syndrome, urea cycle disorder, fibrotic lung diseases, hepatic encephalopathies, thioredoxin mediated diseases, human papilloma virus (HPV) infections, autoimmune diseases, thalassemia, genetic disorders; or from a dis-regulation of HDAC6 activity, the dis-modulation of stem cells, having or threatened by grey hair, the lack of neuronal growth, having or threatened by bone and joint diseases, or subject to cancer therapy by applying a suitable amount of at least one a Phenylbutyl-Derivative of Formula I (or of Formula II) according to the invention to the being. Preferably this application is done in form of a medicament or pharmaceutical formulation.

For the medicament or pharmaceutical formulation according to the present invention or for a medicament or pharmaceutical formulation used according to the invention the medicament or pharmaceutical formulation can be of any form suitable for the application to humans and/or animals, preferably humans including infants, children and adults and can be produced by standard procedures known to those skilled in the art. The medicament or pharmaceutical formulation can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The medicament or pharmaceutical formulation of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments or pharmaceutical formulation may for example be injected intramuscularly, intraperitoneally, or intravenously.

Medicaments or pharmaceutical formulation according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or modified or controlled release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments or pharmaceutical formulation according to the present invention may also comprise an enteric coating, so that their dissolution is dependent on pH-value. Due to said coating the medicament may pass the stomach undissolved and the respective Phenylbutyl-Derivative of Formula I (or of Formula II) according to the invention is liberated in the intestinal tract. Preferably the enteric coating is soluble at a pH value of 5 to 7.5. Suitable materials and methods for the preparation are known from the prior art.

Typically, the medicaments according to the present invention may contain 1-60 % by weight of one or more of the Phenylbutyl-Derivatives of Formula I (or of Formula II) according to the invention and 40-99 % by weight of one or more auxiliary substances (additives/excipients).

The joint mixture of the present invention may also be administered topically or via a suppository.

The daily dosage for humans and animals may vary depending on factors that have their basis in the respective species or other factors, such as age, sex, weight or degree of illness and so forth. The daily dosage for humans preferably is in the range of 10 to 2000 milligrams of active substance (Phenylbutyl-Derivative of Formula I (or of Formula II) according to the invention) to be administered during one or several intakes per day.

In a very preferred embodiment the medicament or pharmaceutical formulation of the invention is an oral pharmaceutical composition. Very suitable oral pharmaceutical compositions comprising 4-phenyl-butyric acid or a physiologically acceptable salt thereof are disclosed in EP1427396 (WO2003/22253), the content of which forming herewith part of the description/disclosure of the current invention. Accordingly, it is very preferred if such an oral pharmaceutical composition comprising a Phenylbutyl-Derivative of Formula I (or of Formula II) according to the invention is formulated in an analoguous way to the formulation presented in EP1427396 (WO2003/22253). It is thus further very preferred if an oral medicament according to the invention especially a pharmaceutical composition comprises 100 to 2000 mg of the Phenylbutyl-Derivative of Formula I (or of Formula II) according to the invention, preferably 200 to 1000 mg, most preferably approximately 500 mg or 250 mg of the Phenylbutyl-Derivative of Formula I (or of Formula II) according to the invention.

In another alternative embodiment the Phenylbutyl-Derivative of Formula I (or of Formula II) according to the invention is formulated in a medicament or pharmaceutical formulation in form of a tablet wherein each gram of granulate contains 100 to 2000 mg of the Phenylbutyl-Derivative of Formula I (or of Formula II) according to the invention, preferably 500 to 1000 mg of the Phenylbutyl-Derivative of Formula I (or of Formula II) according to the invention. Preferably, the granulate does further comprise microcrystalline cellulose, magnesium stearate and/or colloidal anhydrous silica.

In another alternative embodiment the Phenylbutyl-Derivative of Formula I (or of Formula II) according to the invention is formulated in a medicament or pharmaceutical formulation in form of a granulate containing 100 to 2000 mg of the Phenylbutyl-Derivative of Formula I (or of Formula II) according to the invention, preferably 500 to 1000 mg of the Phenylbutyl-Derivative of Formula I (or of Formula II) according to the invention. Preferably the granulate does further comprise calcium stearate and/or colloidal anhydrous silica.

Another aspect of the invention refers to a process for producing a Phenylbutyl-Derivative of Formula I according to the invention, wherein in one part of the process a compound of Formula III 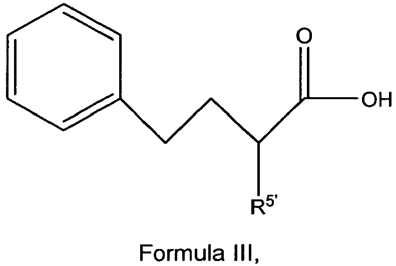 wherein R^{5'} is either hydrogen or 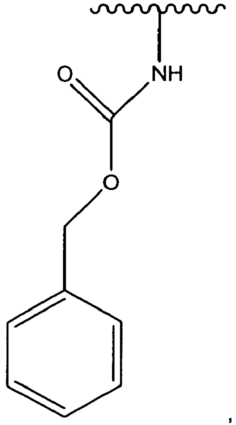 is reacted with a compound of either - if n is ≠ 0 - Formula IV 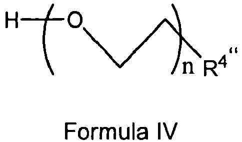 or - if n is 0 - R⁴, with R⁴' being selected from a salicylic acid derivative, a radical of an amino acid or of 4-phenyl butyric acid either as free acid, or base or salt thereof,
wherein R⁴" and R⁴' optionally comprises one or more covalently bound additional amino acids and/or optionally one or more protecting groups PG and/or optionally one or more leaving groups LG like a halogen.

The present invention is illustrated below with the help of figures and examples. These illustrations are given solely by way of example and do not limit the invention.

### FIGURES:

- **Figures 1A/B:**: Example 9: Effects of Staurosporine (STS) on cell proliferation and apoptosis.
- **Figures 2A/B:**: Example 9: Effects of EXAMPLE 3 on cell proliferation and apoptosis.
- **Figures 3A/B:**: Example 9: Effects of EXAMPLE 4 on cell proliferation and apoptosis.
- **Figures 4A/B:**: Example 9: Effects of 4-phenylbutyrate (4-PB) on cell proliferation and apoptosis.
- **Figures 5A/B:**: Example 9: Effects of DMSO concentration on cell proliferation and apoptosis
- **Figures 6A/B:**: Example 9: Effects of Staurosporine (STS) on cell proliferation and apoptosis in presence of 100µM zVAD.
- **Figures 7A/B:**: Example 9: Effects of Example 3 on cell proliferation and apoptosis in presence of 100µM zVAD.
- **Figures 8A/B:**: Example 9: Effects of Example 4 on cell proliferation and apoptosis in presence of 100µM zVAD.
- **Figures 9A/B:**: Example 9: Effects of 4-phenylbutyrate (4-PB) on cell proliferation and apoptosis in presence of 100µM zVAD.
- **Figure 10:**: Synthesis Scheme covering the Synthesis of Examples 1 and 2.
- **Figure 11:**: Synthesis Scheme covering the Synthesis of Examples 3 and 4.
- **Figure 12:**: ¹H-NMR of Example 3 in CDCl3.
- **Figure 13:**: ¹H-NMR of Example 4 in CDCl3.

### EXAMPLES:

### Example 1: Synthesis of 2-(4-Phenyl-butyryloxy)-benzoic acid (Example 1)

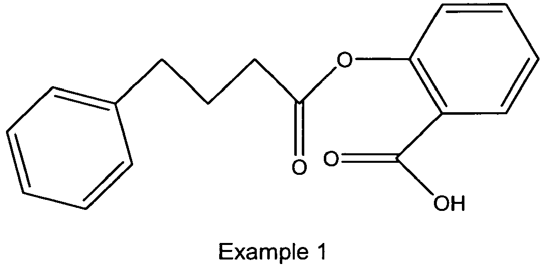

### 1 st Step:

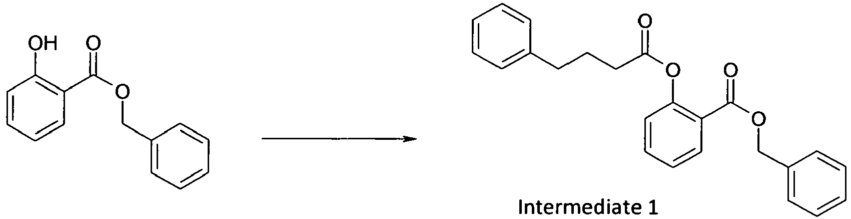

To a solution of Benzylsalicylat (2.74g) in DCM (40ml) and NEt3 (3.33ml) was added 4-Phenylbutanoylchloride (3g) dissolved in 15 ml DCM in an ice bath. The mixture was stirred for 18 h at 0°. After dilution with DCM the organic Phase was washed with 1 N HCl, 10% Na2CO3 solution and satd. NaCl solution. The organic layer was dried with NaSO4 and concentrated under reduced pressure. The residue was crystallized from Ethanol. I**ntermediate 1** was obtained as a colorless solid (4.36g, 11.5mmol, 96%).

1 H-NMR in CDCl3
8.06 (dd, j=1.6, 7.8, 1H), 7.55 (m, 1H), 7.41-7.11 (m,11H), 7.05(dd, j=1.0, 8.1, 1H), 5.30(s, 2H), 2.68, (m,2H), 2.45 (m,2H), 1.95 (m,2H). M+Na= 397.

### 2^{nd} Step:

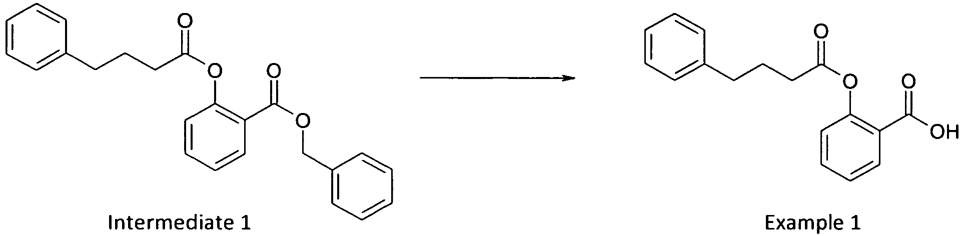

A suspension of **Intermediate 1** (4.3g) and Pd/C (5%Pd) (430mg) in THF (43ml) was stirred under H2-atmosphere. After completion of the reaction, the Pd/C was removed by filtration and was concentrated under reduced pressure. The crude product was treated with heptane. The crystalline solid was separated by filtration and leaded to **Example 1** (2.53g, 8.9 mmol, 77%).

1H-NMR in CDCl3
8.10 (dd, j=1.6, 7.8, 1H), 7.6 (m, 1H), 7.41-7.11 (m, 6H), 7.10(dd, j=1.0, 8.1, 1H), 2.75 (m, 2H), 2.65 (m, 2H), 2.11 (m, 2H). M+Na= 307.

### Example 2: Synthesis of 2-(2-Amino-4-phenyl-butyryloxy)-benzoic acid (Example 2)

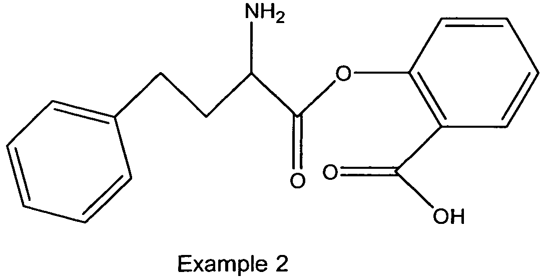

### 1^{st} Step:

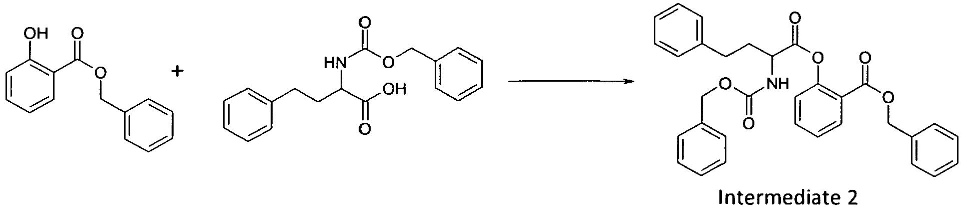

A solution of Benzylsalicylat (3.2g), Z-hPhe-OH (4.4g), EDCxHCl (3.1g) and DMAP (0.17g) in DCM (53ml) was stirred for 24h.The organic phase was washed with 1 N NaOH, 1N HCl and satd. Na2CO3. The organic layer was dried with NaSO4 and concentrated under reduced pressure. The crude product was treated with heptane to give **Intermediate 2** (6.43g, 12.3mmol, 88 %) as colorless solid, which was separated by filtration.

1H-NMR in CDCl3
8.04 (dd, j=1.7, 6.2, 1H), 7.55 (m, 1H), 7.40-7.15 (m,13H), 7.06 (d, j=8.0, 1H), 5.40 (m, 1H).5.27 (m, 2H), 5.15 (m, 2H), 4.63(m, 1H), 2.78 (m, 2H), 2.41 (m, 1H), 2.11 (m, 1H). M+Na= 546.

### 2^{nd} Step:

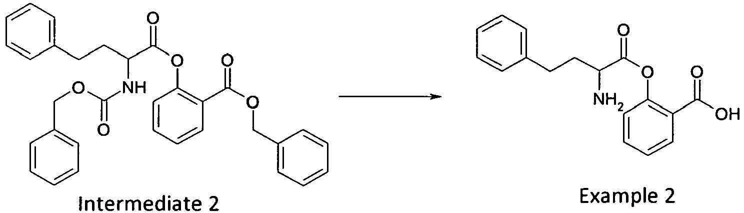

A suspension of **Intermediate 2** (6.4g) and Pd/C (5%Pd) (640mg) in THF (64ml) was stirred under H2-atmosphere. After completion of the reaction, the Pd/C was removed by filtration and was concentrated under reduced pressure. **Example 2** (3.5g, 11.7mmol, 95%) was obtained as brown foam.

1H-NMR in CDCl3
12.5 (bs, 1H), 9.01 (d, j=7.1, 1H), 8.00 (dd, j= 1.2, 7.8, 1H), 7.42 (m, 1H), 7.30-7.15 (m, 8H), 4.41 (m, 1H), 2.70 (m, 2H), 2.15 (m, 2H). M+Na= 322.

### Example 3: Synthesis of 2-(4-Phenyl-butyrylamino)-propionic acid (Example 3)

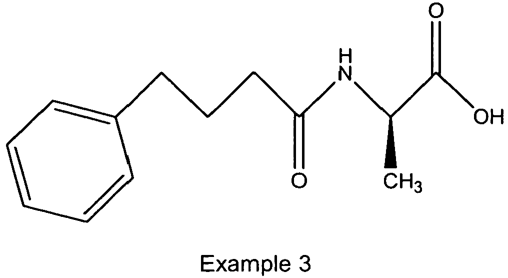

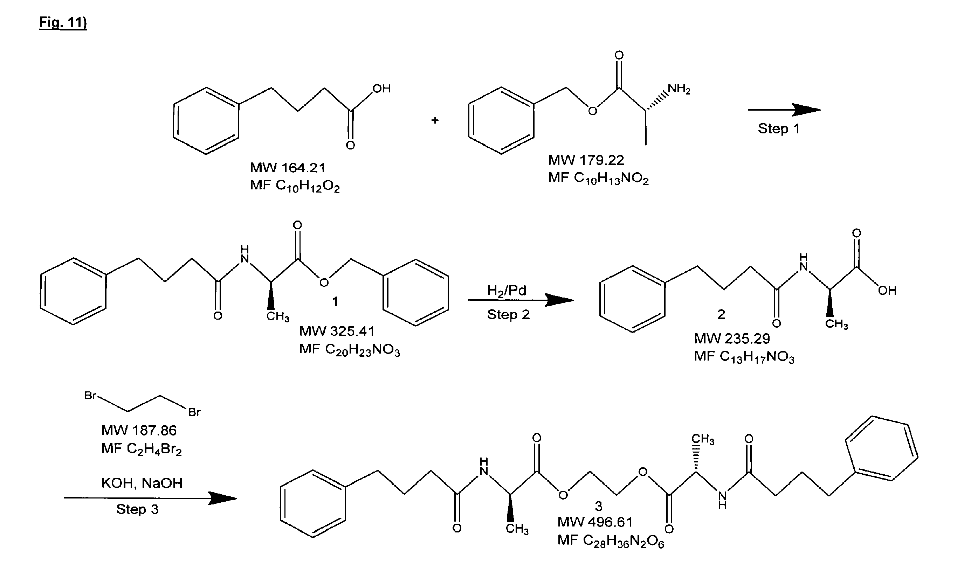

The Synthesis of Example 3 is shown in detail in the Synthesis Scheme of Fig. 11.

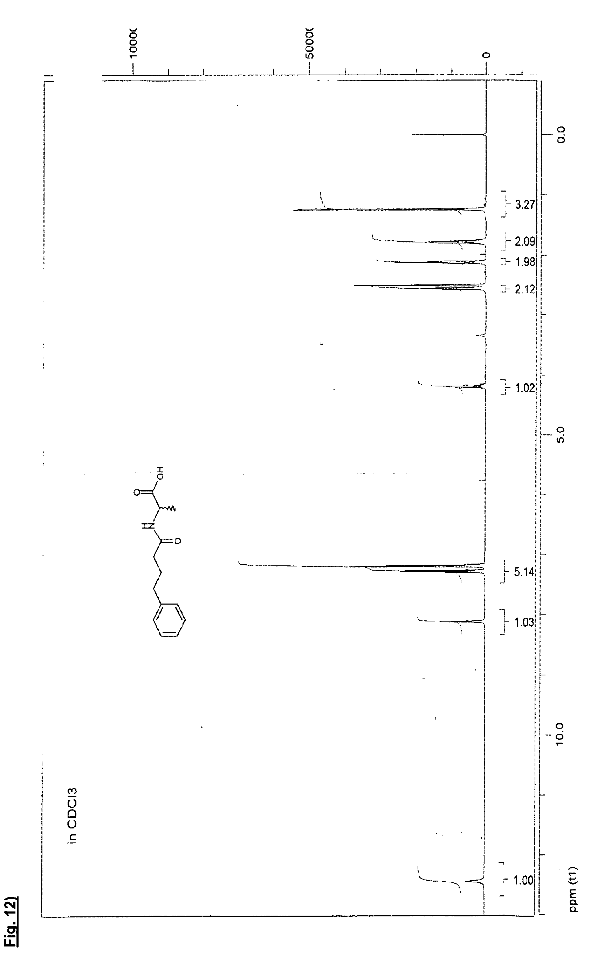

2.20 g of Example 3 were synthesized with a purity (verified by HPLC) of > 97%. The structure was analysed and confirmed by ¹H-NMR in CDCl₃ according to Fig. 12. The ratio of D-Ala to L-Ala was found to be 80% / 20%. Example 3 was soluble in alcohols (ethanol and methanol) as well as in acetic acid. Molecular Weight is 253.29 g/mol.

### Example 4: Synthesis of 2-(4-Phenyl-butyrylamino)-propionic acid 2-[2-(4-phenyl-butyrylamino)-propionyloxyl-ethyl ester (Example 4)

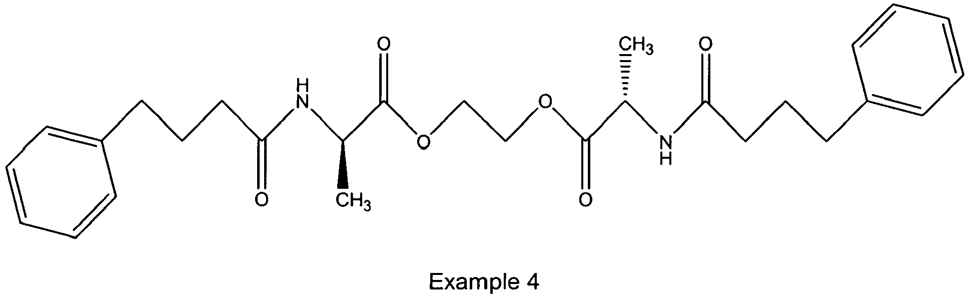

The Synthesis of Example 4 is shown in detail in the Synthesis Scheme of Fig. 11.

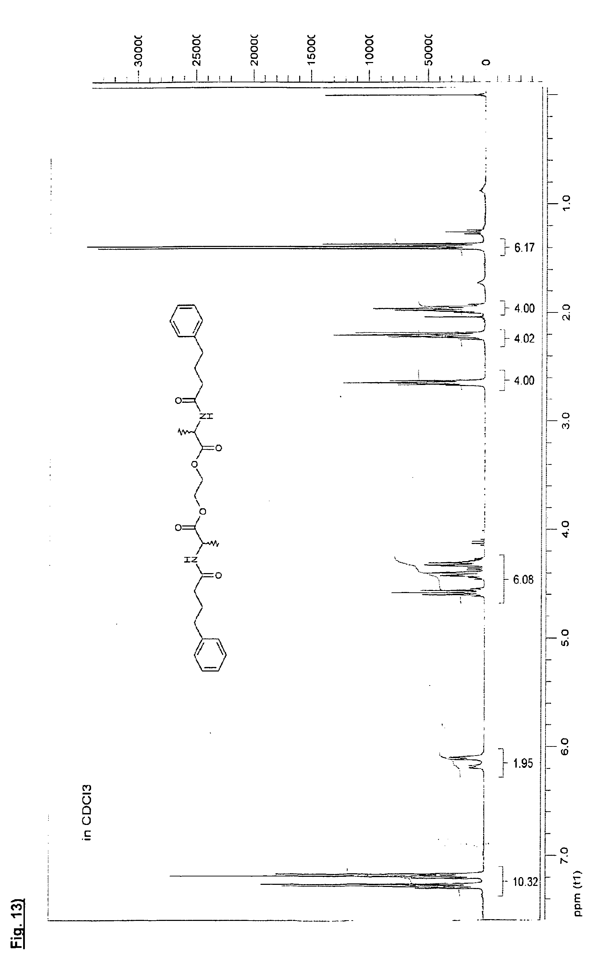

1.67 g of Example 4 were synthesized with a purity (verified by HPLC) of > 97%. The structure was analysed and confirmed by ¹H-NMR in CDCl₃ according to Fig. 13. The ratio of D-Ala to L-Ala was found to be 80% / 20%. Example 4 was soluble in dichloromethane, ethyl acetate and acetonitrile. Molecular Weight is 496.61 g/mol.

### Example 5: Synthesis of 4-Phenyl-butyric acid 2-acetoxy-phenyl ester (Example 5)

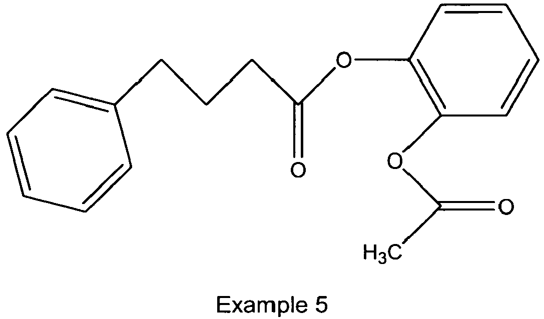

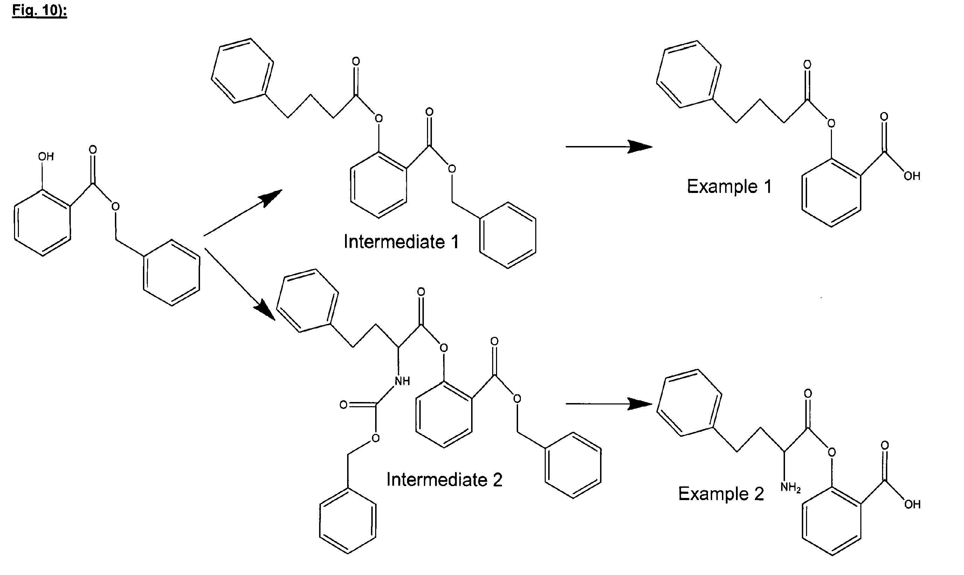

Example 5 is synthesised analogous to the synthesis of Example 1 above (and the Synthesis Scheme of Fig. 10).

### Example 6: Synthesis of 2-Amino-4-phenyl-butyric acid 2-acetoxy-phenyl ester (Example 6)

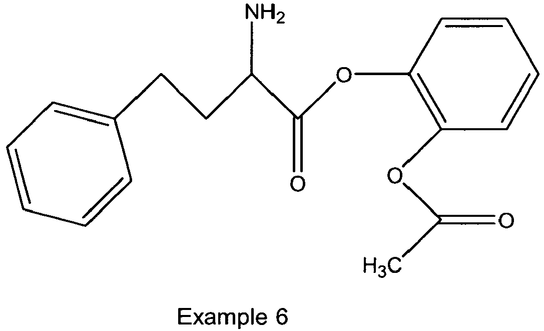

Example 5 is synthesised analogous to the synthesis of Example 2 above (and the Synthesis Scheme of Fig. 10).

### Example 7: Synthesis of 2-Acetoxy-6-(4-phenyl-butyl)-benzoic acid (Example 7)

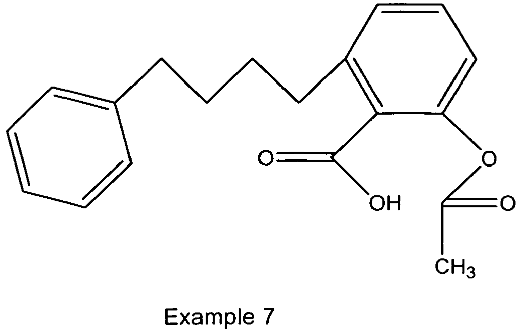

Example 5 is synthesised analogous to the synthesis of Example 2 above (and the Synthesis Scheme of Fig. 10).

### Example 8: Synthesis of 2-Acetoxy-benzoic acid 2-(4-phenyl-butyryloxy)-ethyl ester (Example 8)

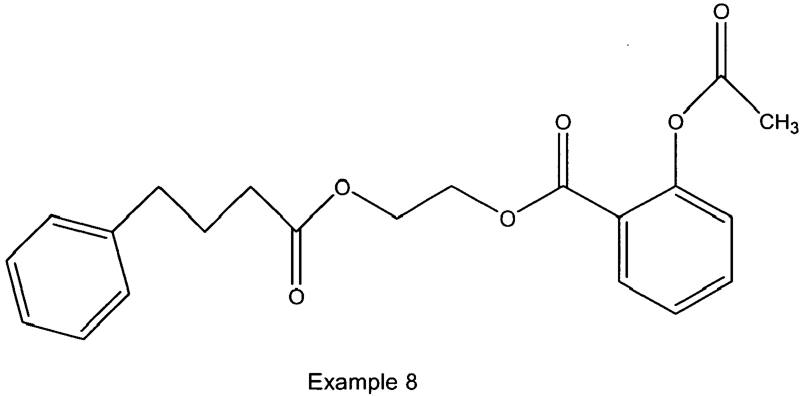

Example 8 is synthesised analogous to the synthesis of Example 1 (and the Synthesis Scheme of Fig. 10).

### Example 9: In vitro effects on apoptosis and proliferation of Jurkat cells tested for the compounds of Examples 3 and 4.

### I. AIM OF THE STUDY

The aim of this study was to test the two compounds of Examples 3 and 4 for their effects on apoptosis and proliferation of Jurkat cells in vitro.
- Study of the effects of the tested compounds on apoptosis and proliferation of Jurkat cells.
- Study of the caspase dependency of cell death induced by compounds of Example 3 and 4 according to the invention in the Jurkat cell line.

### II. STUDY OUTLINE

### A. Biological system

This study was performed using the following cell line on the two types of experiments performed:
- Experiment 1: Study of the effects of compounds of Examples 3 and 4 according to the invention on apoptosis and cell proliferation:
   o Jurkat: acute T cell leukemia
- Experiment 2: Study of the caspase dependency of cell death induced by the compounds of Example 3 and 4 according to the invention.
   o Jurkat: acute T cell leukemia

### B. Test compounds

The following concentrations were set to perform the dose-response studies:

| **Compounds** | **Concentrations tested** |
|---|---|
| Example 3: | 0; 3x10⁻⁵; 10⁻⁴; 3x10⁻⁴; 10⁻³; 3x10⁻³; 10⁻²; 3x10⁻² M |
| Example 4: | 0; 5x10⁻⁶; 1.5x10⁻⁵; 5x10⁻⁵; 1.5x10⁻⁴; 5x10⁻⁴; 1.5x10⁻³; 5x10⁻³ M |

### C. Assay endpoints

### Apoptosis

Apoptosis was quantified at the single cell level by titrating active Caspase 3/7. This assay is based on the strong affinity of the FAM-DEVD-FMK peptide (carboxyfluorescein-labeled fluoromethyl ketone) for the catalytic site of the protease. When added to a cell culture, the FAM-DEVD-FMK probe enters cells and covalently binds to the large subunit of the active caspase 3/7. The bound labelled peptide remains within the cytosolic compartment, while the unbound peptides are drained off from the cell.

The probe emits a green fluorescent signal which allows a direct measure of the amount of active caspase 3/7 present in the cell.

### Cell Proliferation

This assay is based on the dilution rate of a fluorescent membrane marker which is a direct function of cell division. This probe is non toxic and allows the multiplexed detection of other parameters on the same cell population. The assay is performed by loading the cells with a fluorescent phospholipid analogue before plating. It is quickly trapped into the cell membranes, and does not exchange anymore with surrounding medium or neighbour cells. Subsequently, Flow cytometry analyzes the dilution rate of the fluorescent probe at the single cell level which is a direct readout of the number of cell divisions.

All the conditions were performed in triplicate.

### D. Experimental procedure

Detection of cellular events was performed in triplicate in 96 well-plates after 48 hours of treatment by automated flow cytometry. Non-linear regressions and IC50 calculation were performed with GraphPad Prism 4.01 software.

Briefly the time course of the experiments was the following:
o day 1 : seeding of cells in 96 well-plates.
o day 2 : treatment with compounds.
o day 4 : analysis at 48h-treatment.

Experiment 1: Effects of the two compounds of Examples 3 and 4 on apoptosis and cell proliferation in the Jurkat cell line:
- The cells were labeled for subsequent cell proliferation measurements and plated in 96 well culture plates in standard culture conditions.
- 24 hours after plating, the cells were treated with the two compounds of Examples 3 and 4.

Experiment 2: Caspase dependency of cell death induced by the two compounds:
- The cells were labeled for subsequent cell proliferation measurements and plated in 96 well culture plates in standard culture conditions.
- After 24 H of culture in standard conditions, the cells were treated with staurosporine (pro-apoptotic reference compound) in dose response (7 concentrations + 0) in the presence or not of one concentration of zVAD-FMK (100µM) a reference pan caspase inhibitor.
- For the test compounds, after 24 H of culture in standard conditions, cells were treated with one concentration of zVAD-FMK (100µM) and the two compounds of Examples 3 and 4 at the determined concentrations.
- 4-PB was also tested in the same experiment to allow direct comparison with Example 3 and Example 4.

zVad-FMK is a cell-permeant pan caspase inhibitor that irreversibly binds to the catalytic site of caspase proteases and can inhibit induction of apoptosis.

### RESULTS

### A. Effects of the two compounds of Example 3 and 4 on apoptosis and proliferation in Jurkat cell line

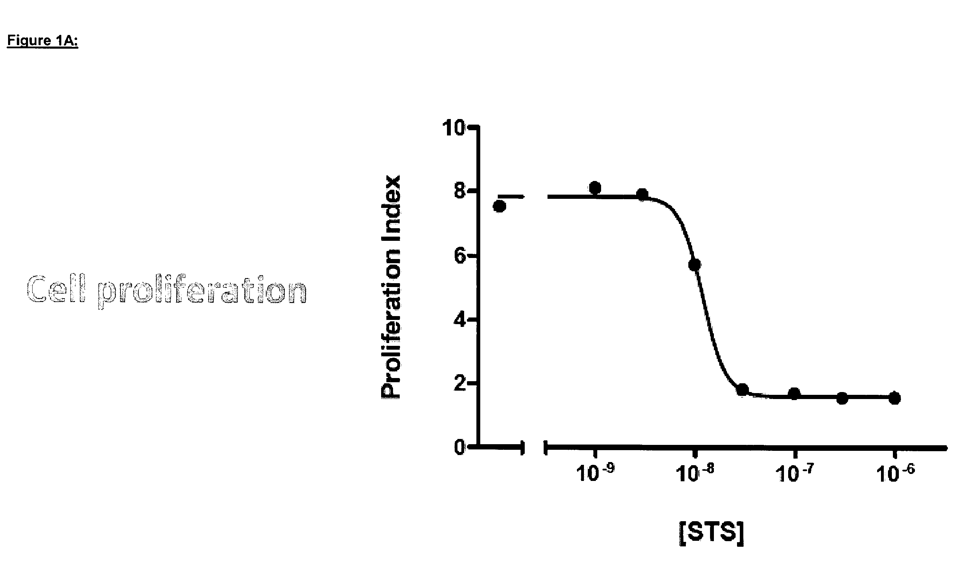

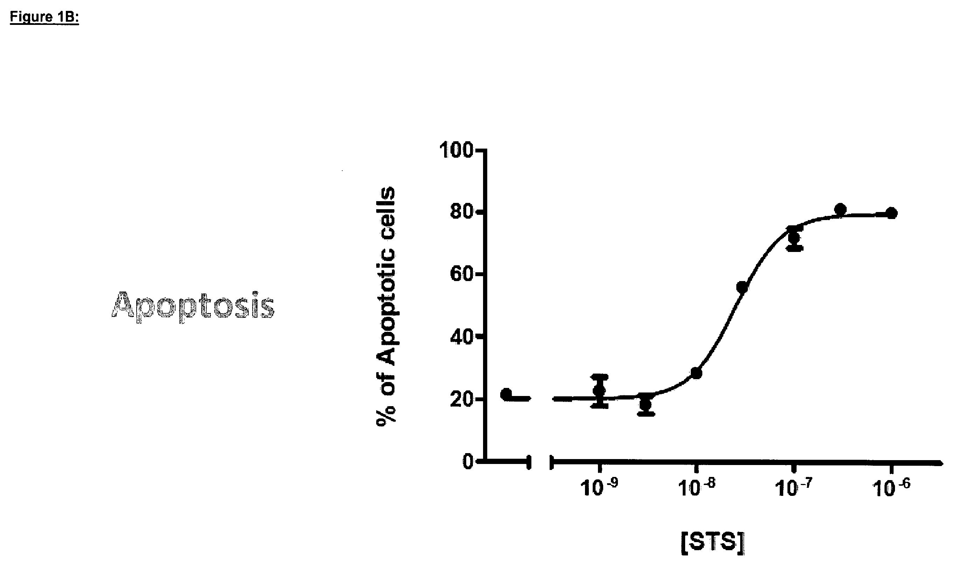

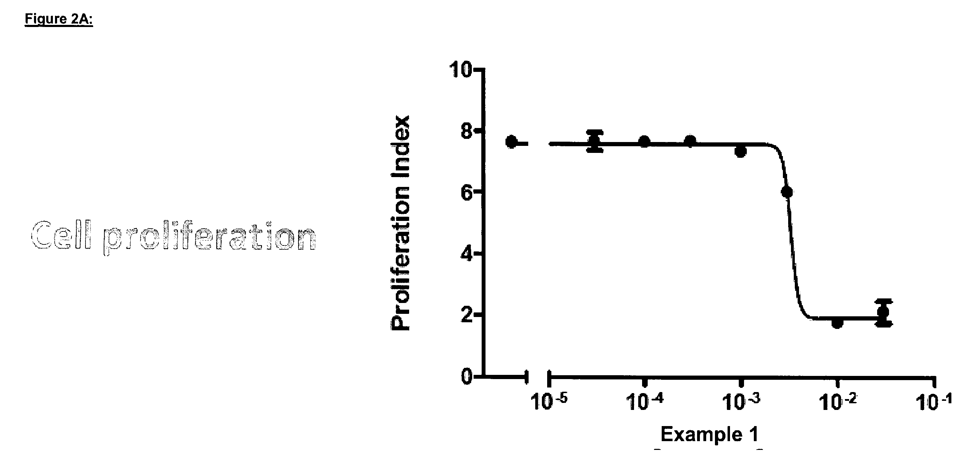

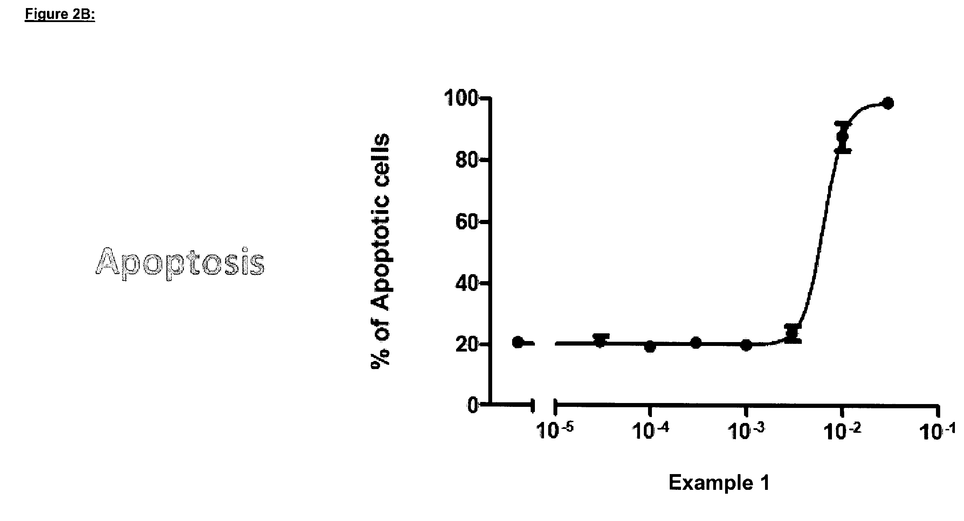

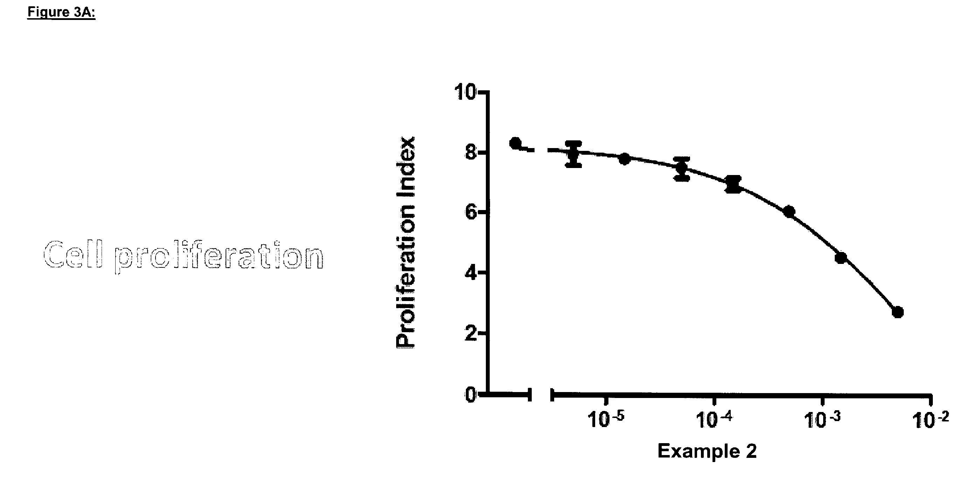

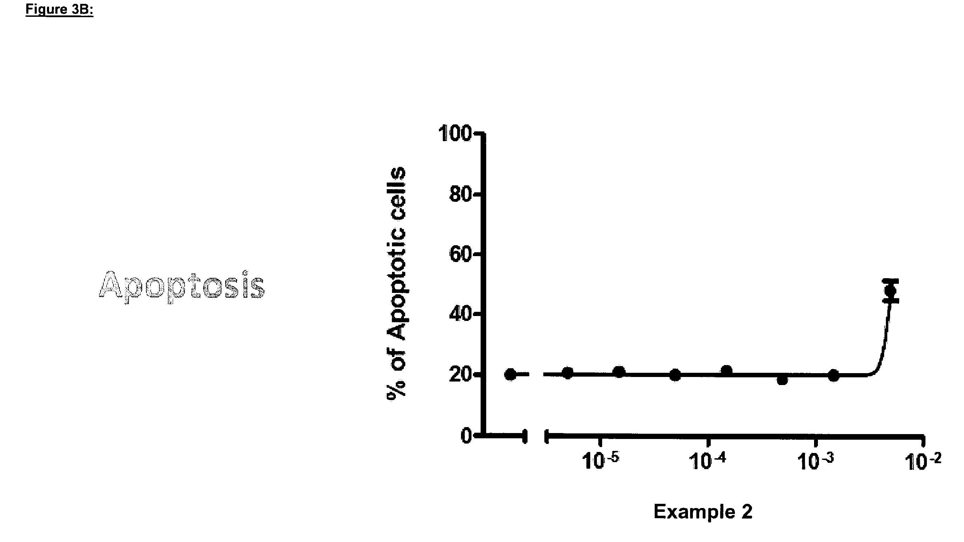

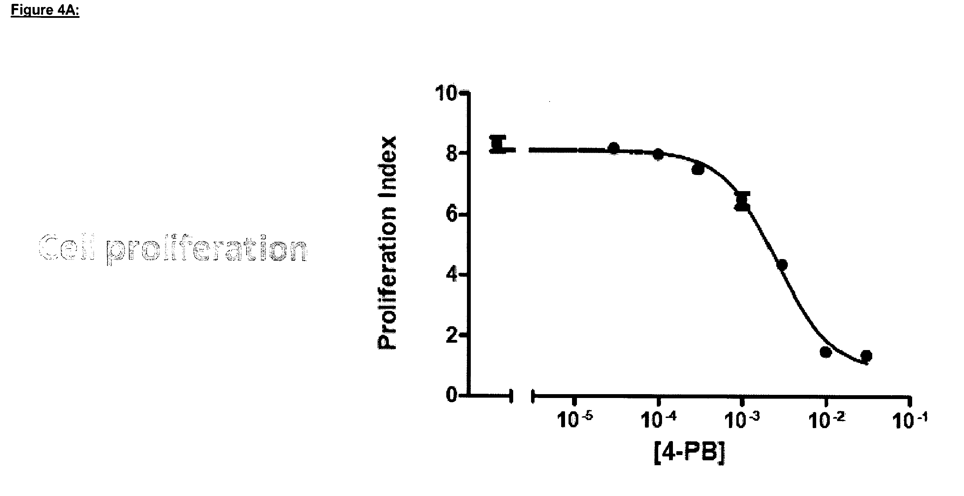

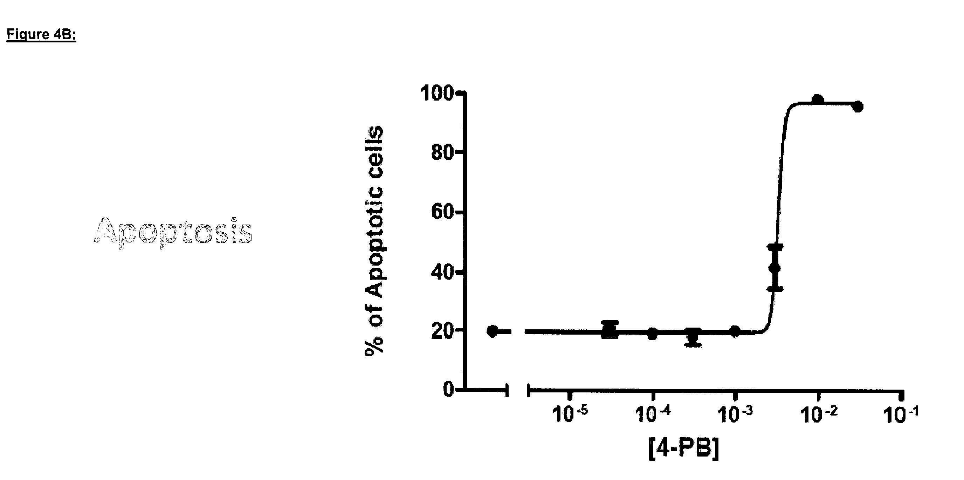

The results of the tested compounds on apoptosis and proliferation in Jurkat cell line can be seen in the figures 1 to 4:
- The effects of Staurosporine (STS) on cell proliferation and apoptosis in figure 1.
- The effects of EXAMPLE 3 on cell proliferation and apoptosis in figure 2.
- The effects of EXAMPLE 4 on cell proliferation and apoptosis in figure 3.
- The effects of 4-phenylbutyrate (4-PB) on cell proliferation and apoptosis in figure 4.

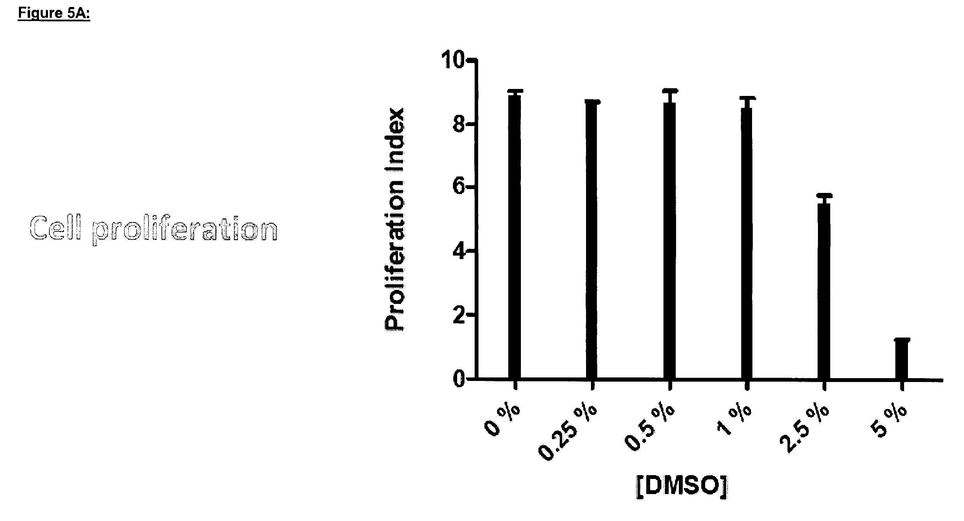

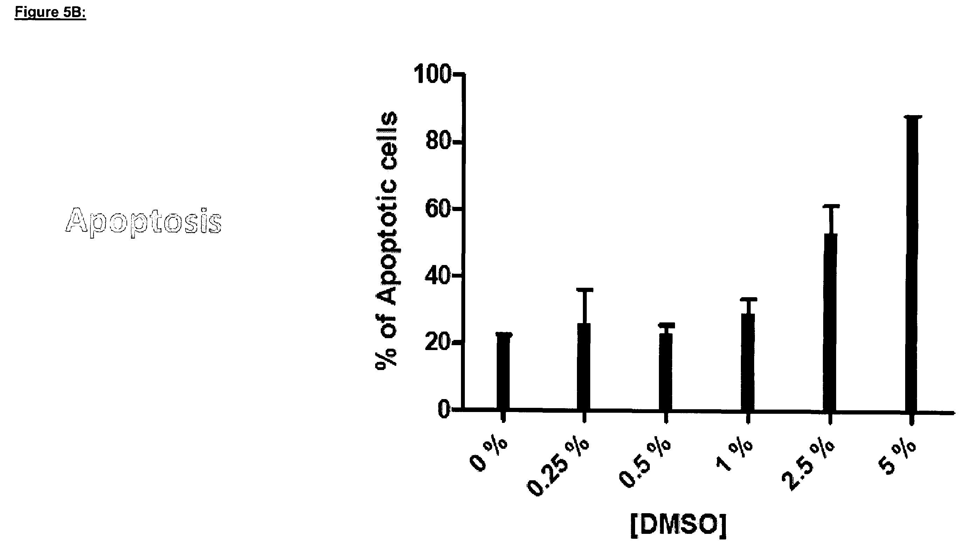

Example 4 stock solution (500mM) was made using DMSO as solvent, because of solubilization issues. Final DMSO concentration in the culture media was 1%. Different final concentrations of DMSO were tested for their impact on proliferation and apoptosis in Jurkat cells. This study revealed that 1% is the maximum tolerable DMSO that can be used as vcan be seen in Figure 5 which shows the effects of DMSO concentration on cell proliferation and apoptosis.

### B. Caspase dependence of the cell death induced by the compounds of Examples 3 and 4 in the Jurkat cell line

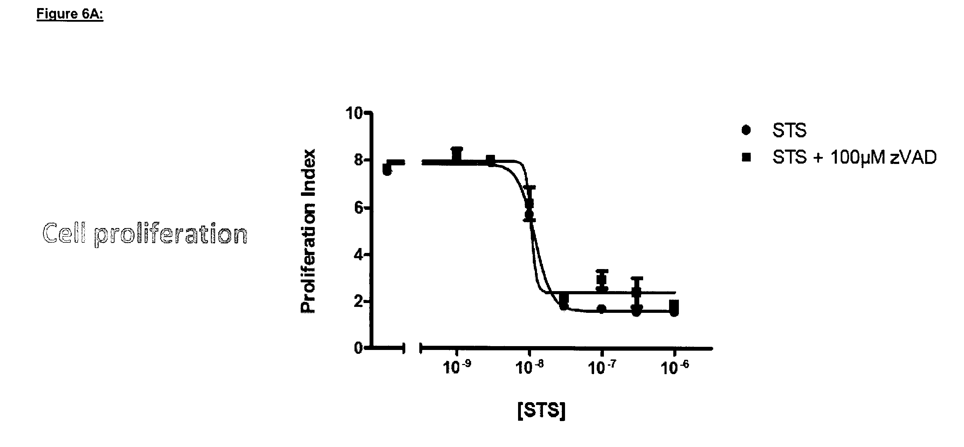

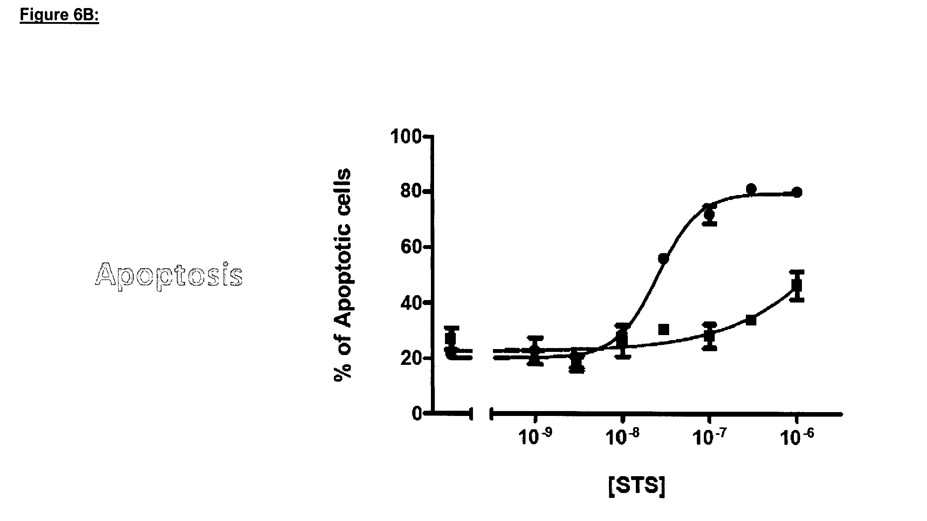

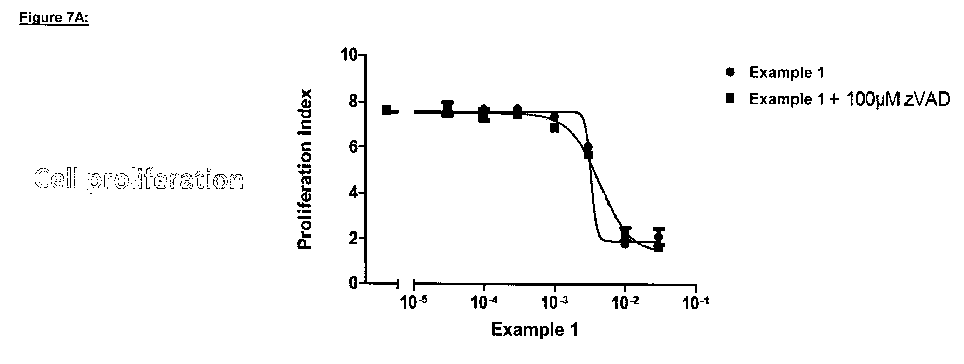

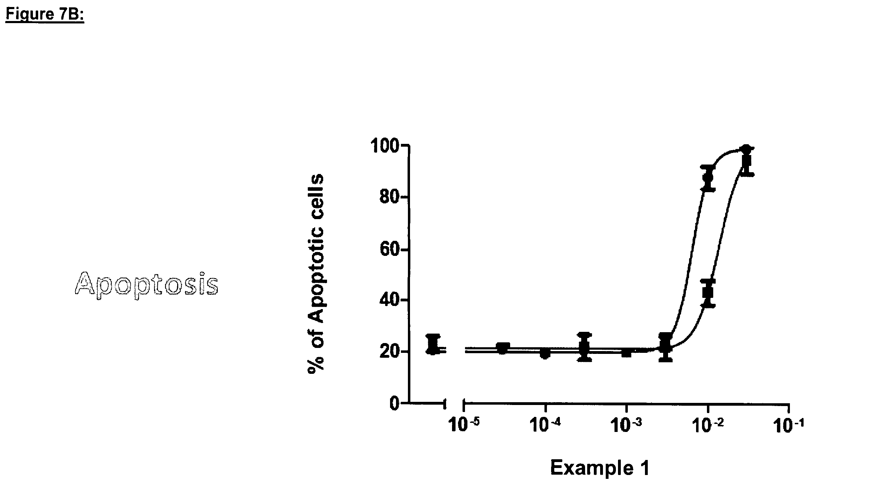

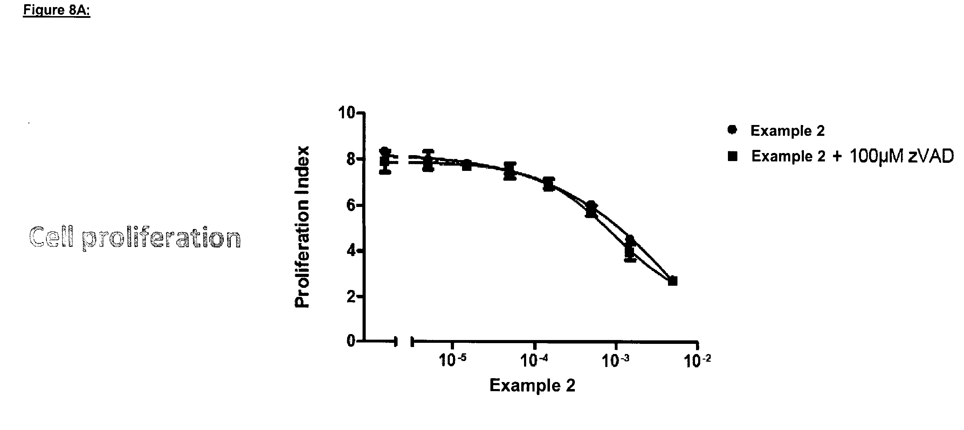

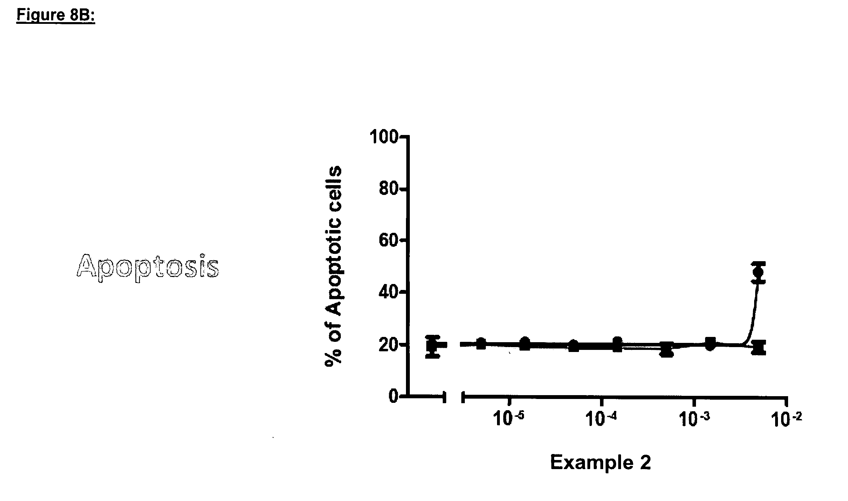

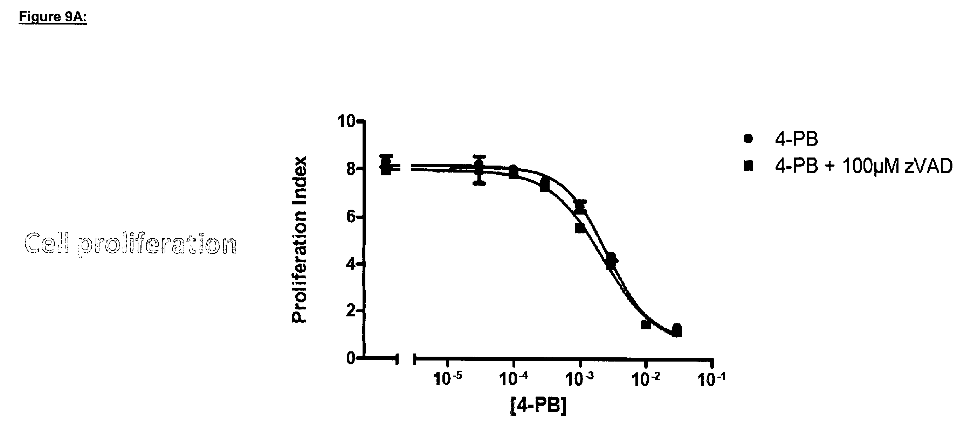

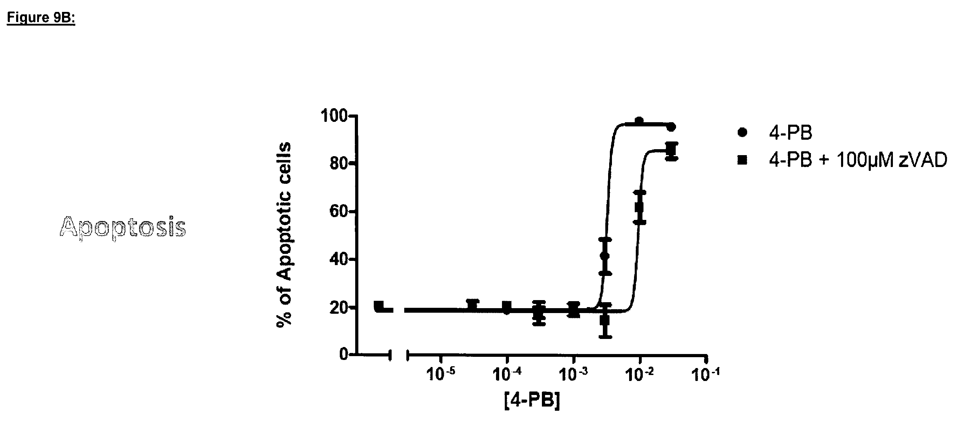

The results of the test on caspase dependence of the cell death induced by the tested compounds in Jurkat cell line can be seen in the figures 6 to 9:
- The effects on caspase dependence of the cell death induced by Staurosporine (STS) in figure 6.
- The effects on caspase dependence of the cell death induced by EXAMPLE 3 in figure 7.
- The effects on caspase dependence of the cell death induced by EXAMPLE 4 in figure 8.
- The effects on caspase dependence of the cell death induced by 4-phenylbutyrate (4-PB) in figure 9.

### IV. CONCLUSIONS

Quality of the test was high according to the Z-factor value of 0,86. The calculation of the Z-factor was made using Staurosporine as control.

### 4-PB treatments:

4-Phenylbutyric acid was able to elicit apoptosis and inhibit proliferation in the Jurkat cell line.

Effects of 4-PB were well inhibited by zVAD-FMK with a shift of the dose-response curve and inhibition of the apoptotic effects. This indicates a caspase dependence of the pro-apoptotic effects of 4-PB in Jurkat cells.

### Example 3 treatments:

Example 3 exhibited pro-apoptotic and anti-proliferative effects in the Jurkat cell line (30mM and 10mM).

Those effects were sensitive to 100µM zVAD-fmk (shift in the dose-response cf. EC50 data Table 2). This indicates that apotosis induced by the compound is caspase-dependent.

### Example 4 treatments:

Example 4 was able to induce apoptosis only at the highest concentration tested (5mM). In presence of zVad-fmk no apoptosis was observed at this concentration, indicating, again, a caspase-dependent mechanism (note however that no complete dose response effect was obtained for the range of concentrations tested).

Example 4 was also able to elicit a concentration dependent anti-proliferative effect.

Note that Example 4 stock solution (500mM) was made in DMSO because of solubilization issues. Final DMSO concentration in culture media was 1%. This concentration had no effect on apoptosis and cell proliferation in Jurkat cells as shown in figure 5.

**Table 1: IC₅₀ of the compounds according to Examples 3 and 4 for their anti-proliferative effects (with or without zVAD).**

| **Compound - Experiment** | **IC₅₀ (without zVAD)** | **IC₅₀ (+100µM zVAD)** |
|---|---|---|
| STS - Cell Proliferation | 1.2 x 10⁻⁸ M | 1.1 x 10⁻⁸ M |
| 4-PB - Cell Proliferation | 2.6 x 10⁻³ M | 2.2 x 10⁻³ M |
| Example 3 - Cell Proliferation | 3.2 x 10⁻³ M | 4.3 x 10⁻³ M |
| Example 4 - Cell Proliferation | 9 x 10⁻³ M | NOT DETERMINED |

**Table 2: EC₅₀ of the compounds according to Examples 3 and 4 for their pro-apoptotic effects (with or without zVAD).**

| **Compound - Experiment** | **EC₅₀ (without zVAD)** | **EC₅₀ (+100µM zVAD)** |
|---|---|---|
| STS - Apoptosis | 2.3 x 10⁻⁸ M | NOT DETERMINED |
| 4-PB - Apoptosis | 3.2 x 10⁻³ M | 1 x 10⁻² M |
| Example 3 - Apoptosis | 6.4 x 10⁻³ M | 1.3 x 10⁻² M |
| Example 4 - Apoptosis | NOT DETERMINED | NOT DETERMINED |

## Claims

1. Phenylbutyl-Derivative of Formula I 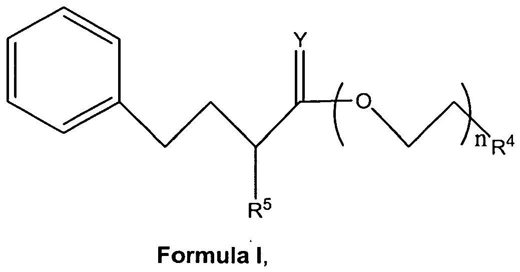 wherein
n is selected from 0, or from 1, 2, 3, 4, 5, 6, 7, 8, 9 and 10;
Y is selected from O or H₂;
R⁵ is selected from H or NH₂; and
R⁴ is selected from either a salicylic acid derivative, a radical of an amino acid or 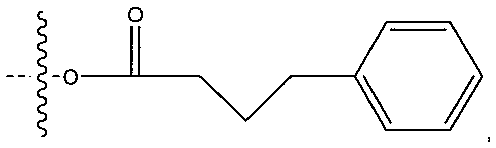 with R⁴ being optionally connected to the core by one or more amino acids;
optionally in form of one of its stereoisomers, enantiomers or diastereomers, its racemate or in form of a mixture of at least two of its stereoisomers in any mixing ratio;
in form of the free acid, free base or salt.

2. Phenylbutyl-Derivative of Formula I according to claim 1, wherein
R⁴ is selected from any of the following structures, which may optionally be substituted by one or more of F, Cl, Br, I, NH₂, OH, SH, CH₃, C₂H₅, C₃H₇, C₄H₉, CH₂Cl, CHCl₂, CF₃, CHF₂, CH₂F: 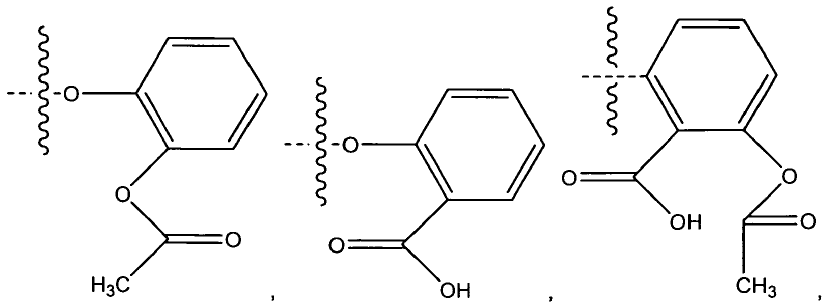 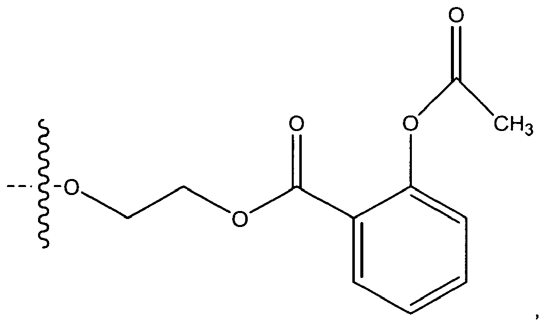 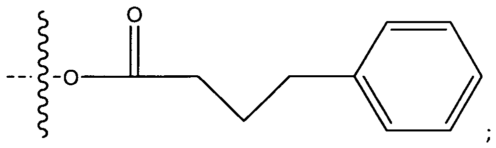 a radical of an amino acid, or
with n ≠ 0 and Y being O 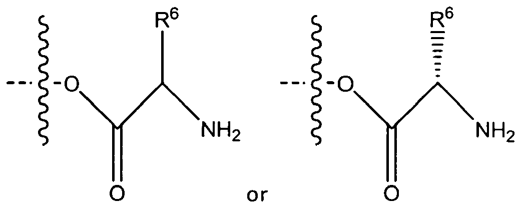 with n being 0 and Y being O

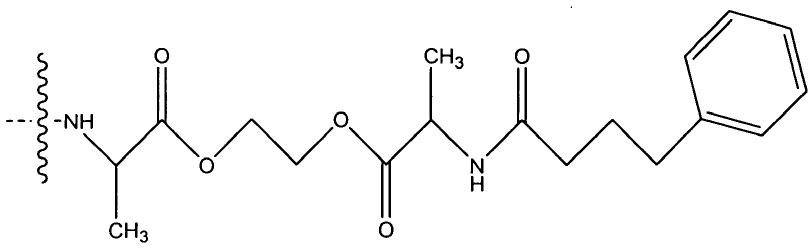

3. Phenylbutyl-Derivative of Formula I according to claims 1 or 2, wherein
R⁴ is selected from any of the following structures 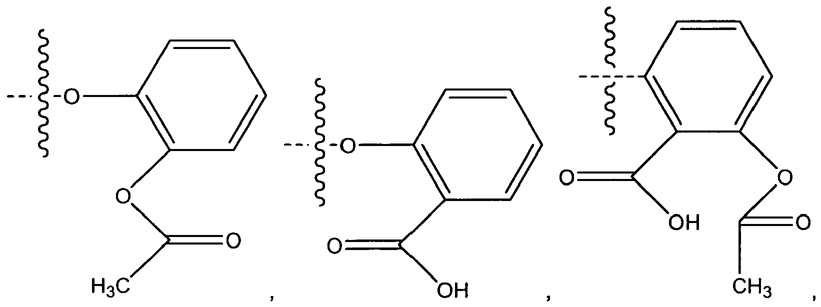 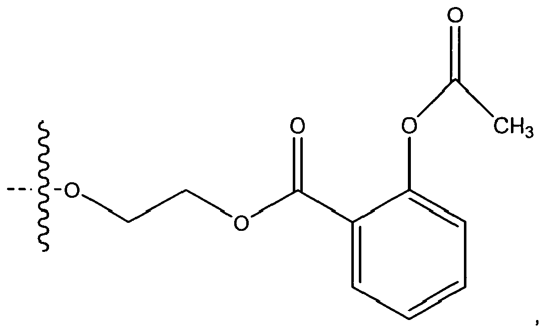 or
a radical of an amino acid, or
with n ≠ 0 and Y being O 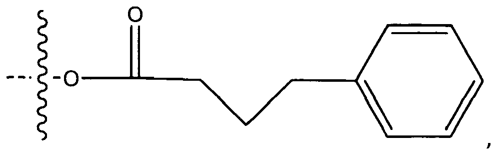 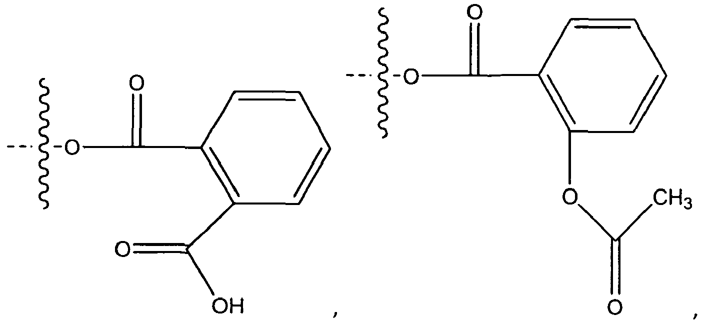 or 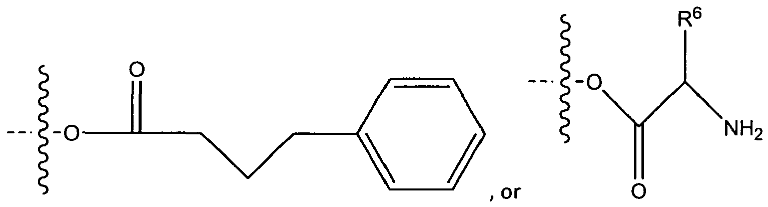 with n being 0 and Y being O 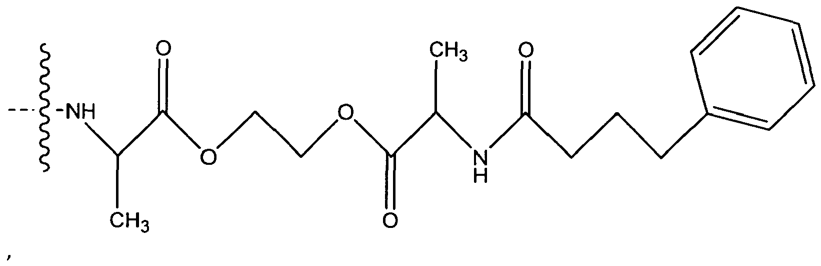 or 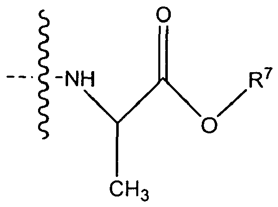 R⁶ being selected from H 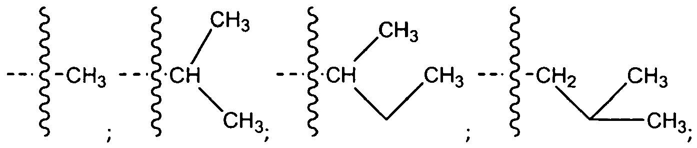 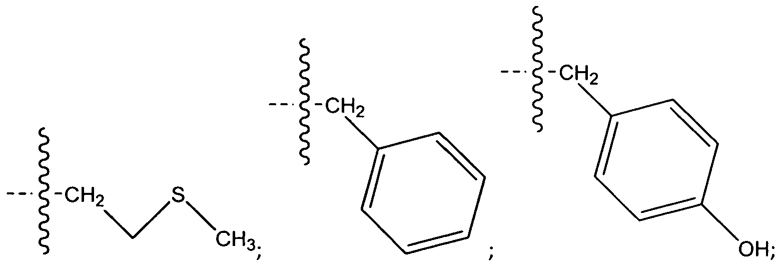 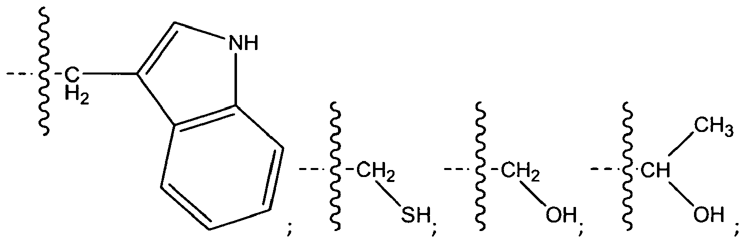 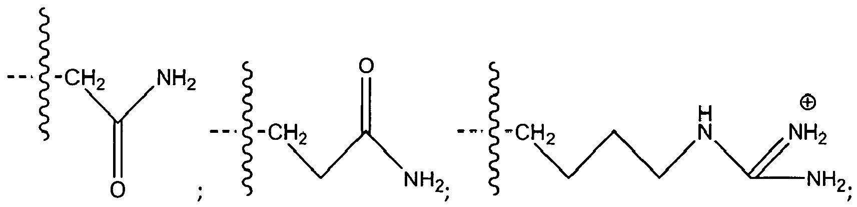 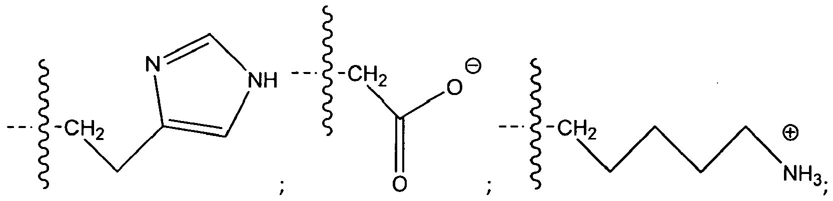 or 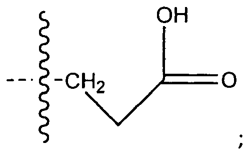 R⁷ being selected from H, C₁₋₄-Alkyl; or 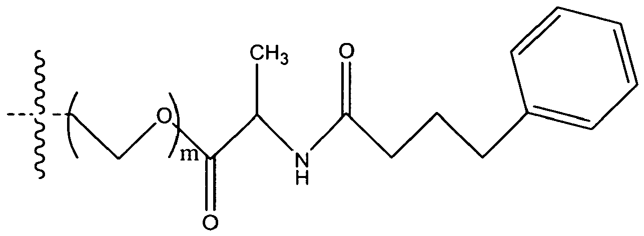 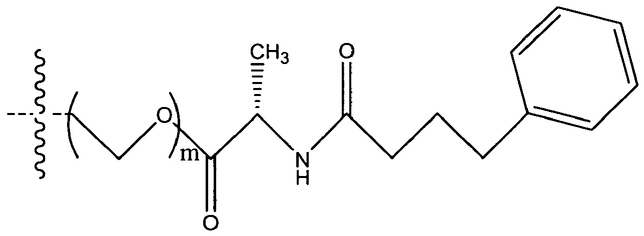 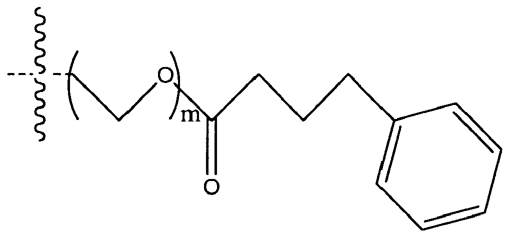 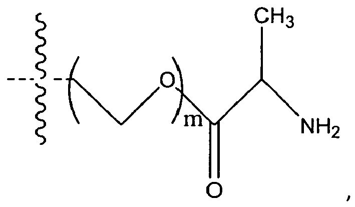 or

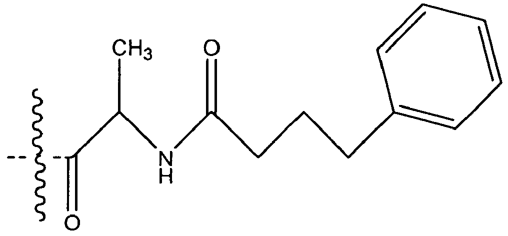

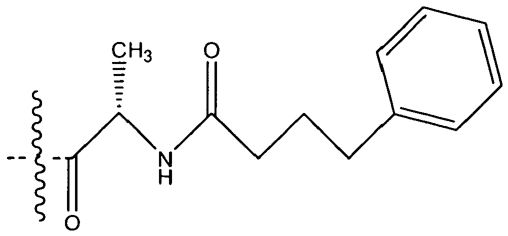

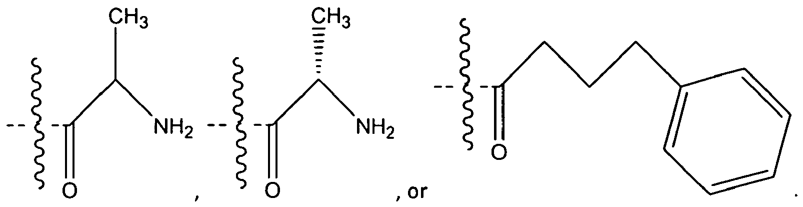

4. Phenylbutyl-Derivative of Formula I according to any of claims 1 to 3, wherein
R⁴ is selected from any of the following structures 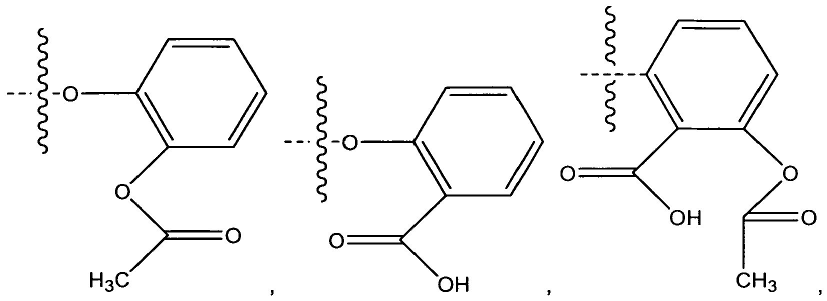 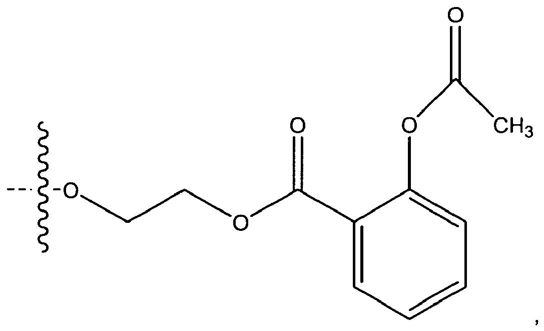 or
a radical of a D-amino acid, or
with n ≠ 0 and Y being O 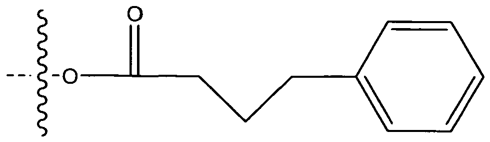 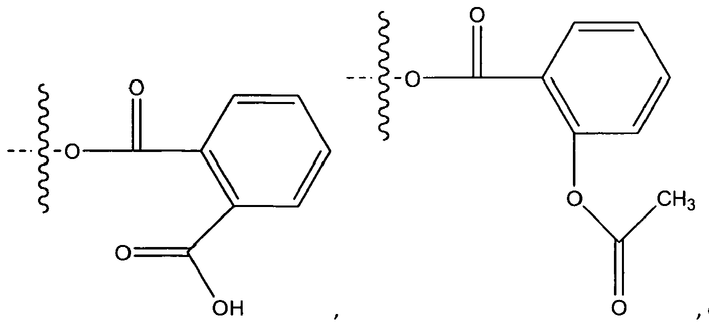 or 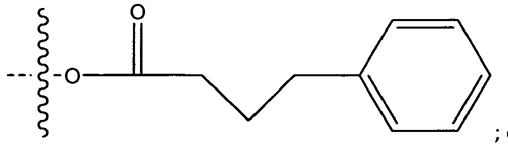 or 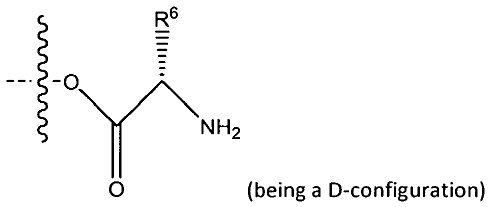 with n being 0 and Y being O 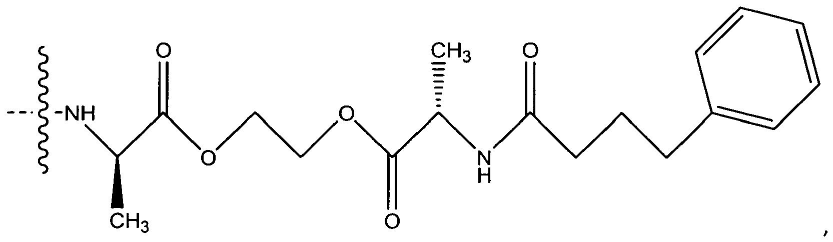 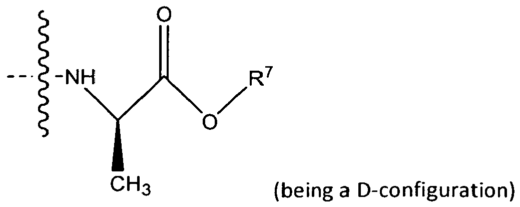 R⁶ being selected from H 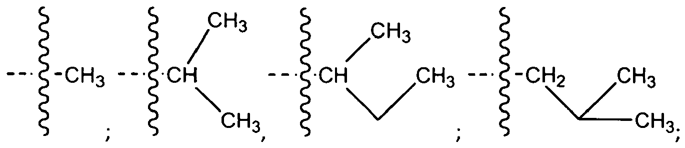 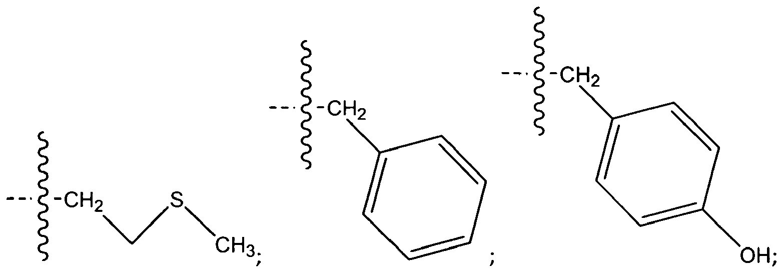 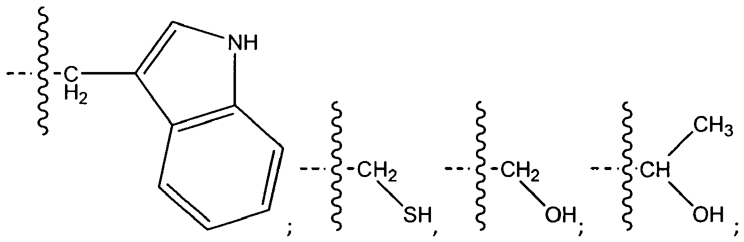 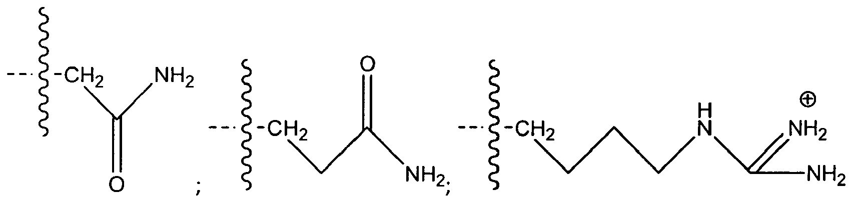 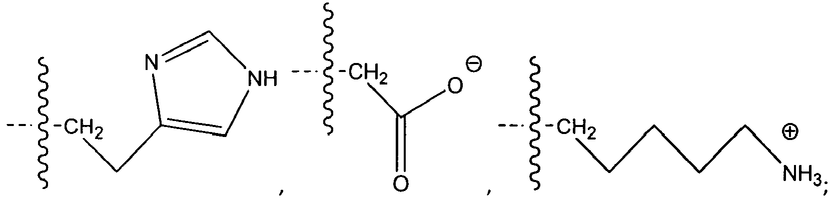 or 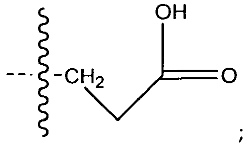 R⁷ being selected from H, C₁₋₄-Alkyl; or

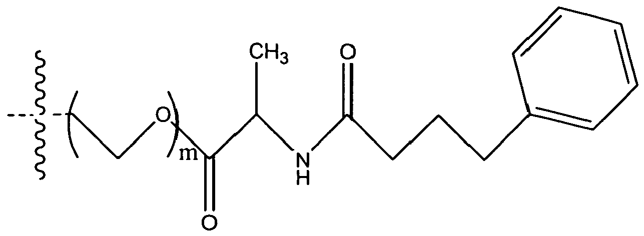

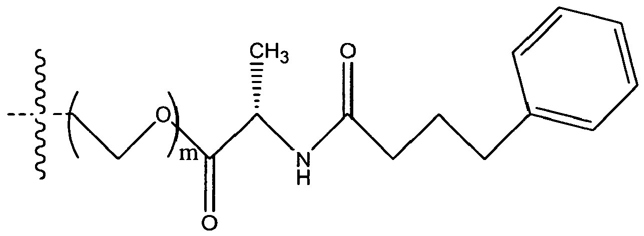

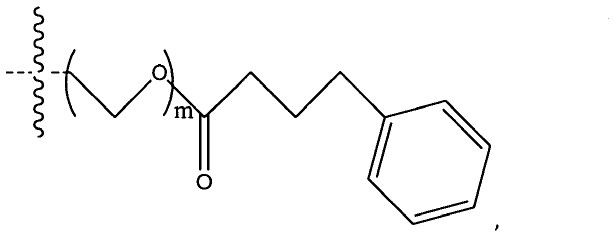

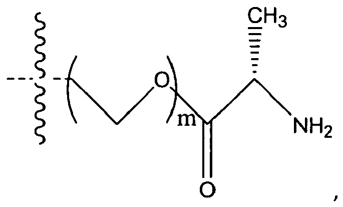

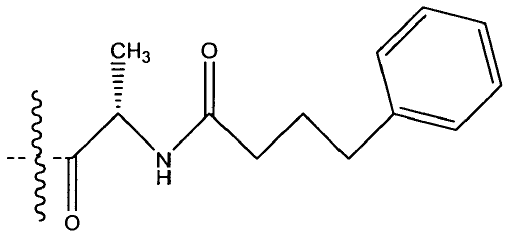

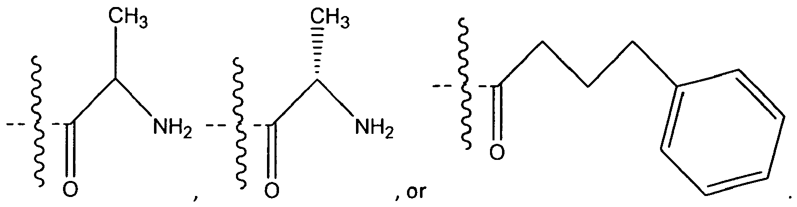

5. Phenylbutyl-Derivative of Formula I according to any of claims 1 to 4, wherein Y is O.

6. Phenylbutyl-Derivative of Formula I according to any of claims 1 to 5, wherein R⁵ is H.

7. Phenylbutyl-Derivative of Formula I according to any of claims 1 to 6, wherein when Y is O and n ≠ 0, R⁴ is selected from any of the following structures 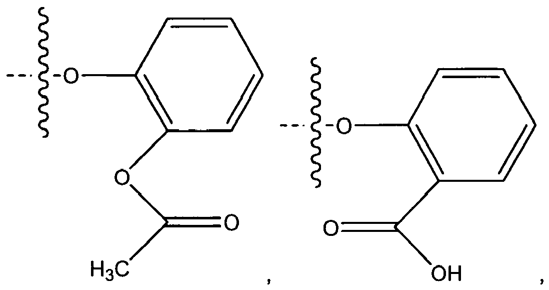 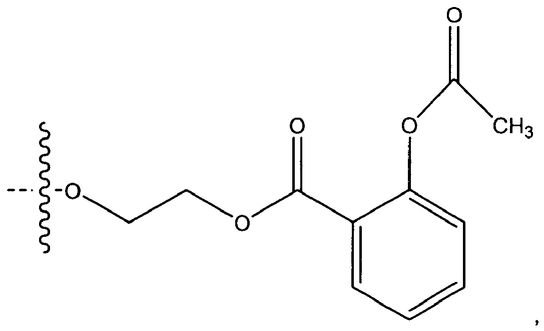 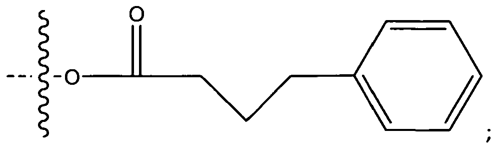 or 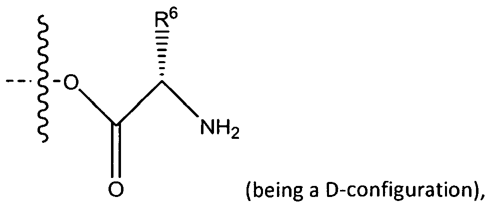 R⁶ being selected from H 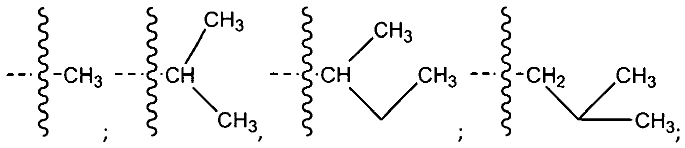 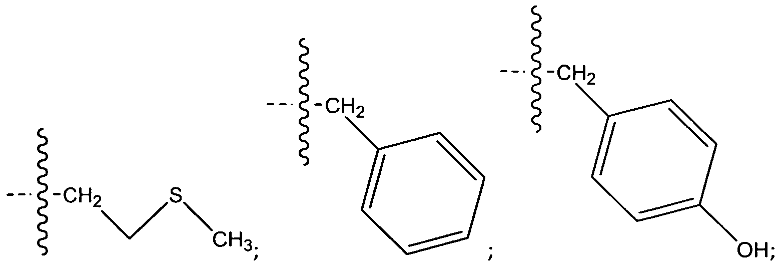 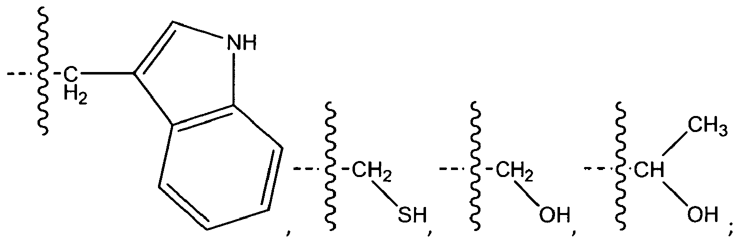 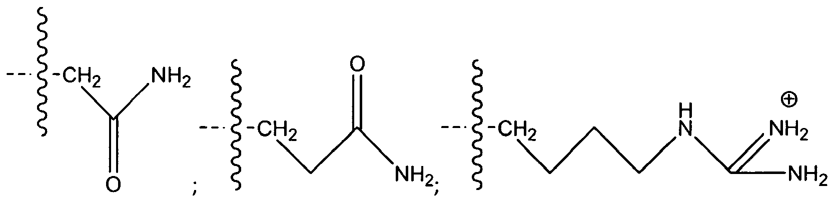 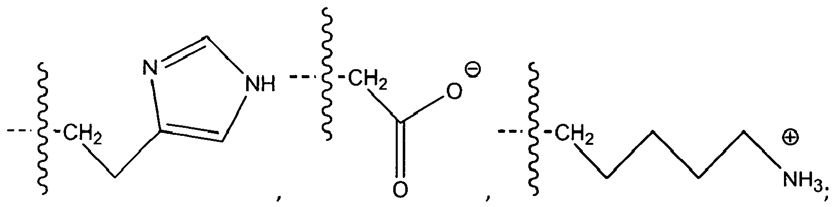 or

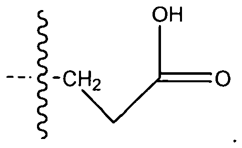

8. Phenylbutyl-Derivative of Formula I according to any of claims 1 to 7, wherein n is 1, 2, 3, 4, 5, or 6, preferably is 1, 2, 3, or 4, or is 1 or 2.

9. Phenylbutyl-Derivative of Formula I according to any of claims 1 to 6, wherein when Y is O and n is 0, R⁴ is selected from any of the following structures 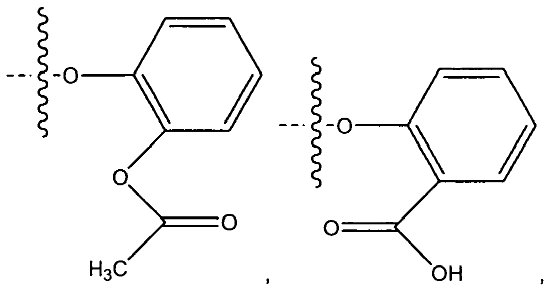 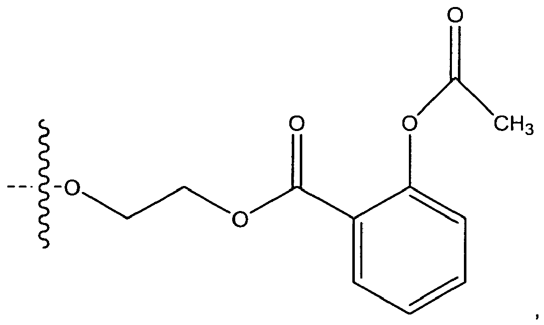 or 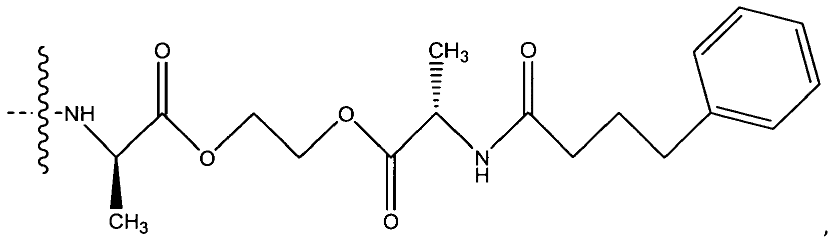 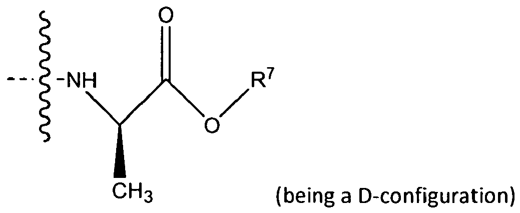 R⁶ being selected from H 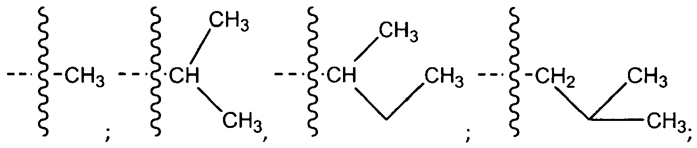 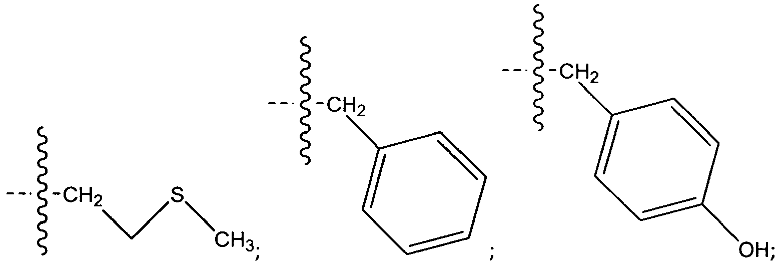 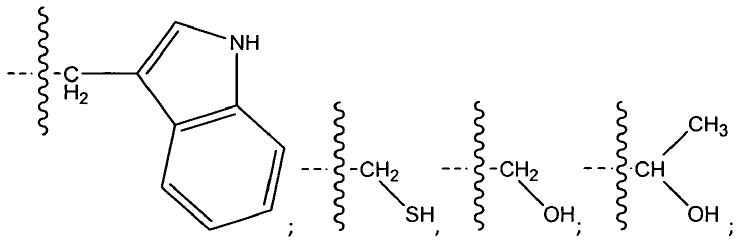 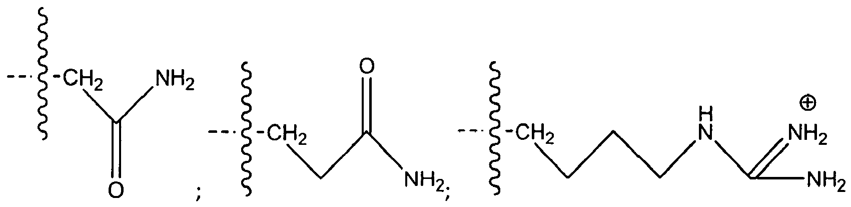 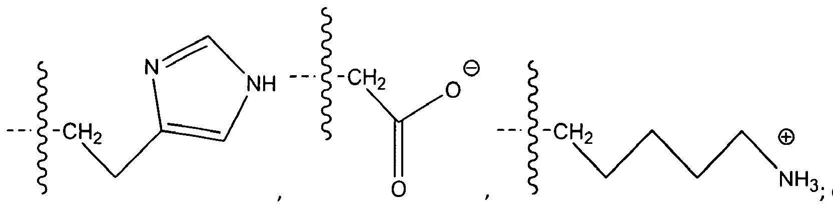 or 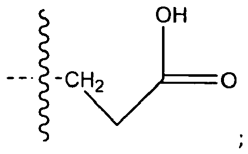 R⁷ being selected from H, C₁₋₄-Alkyl; or 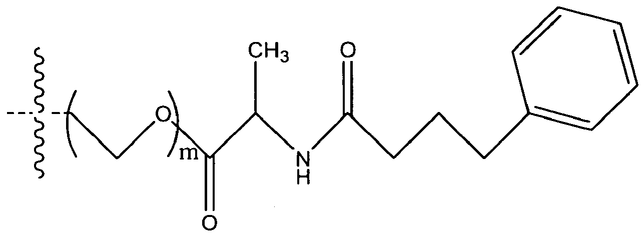 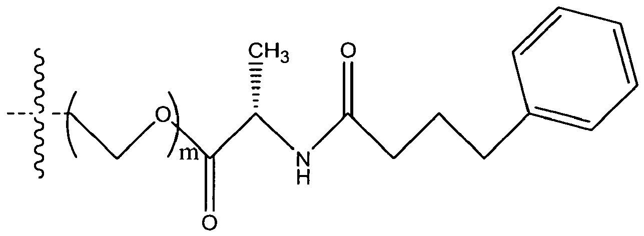 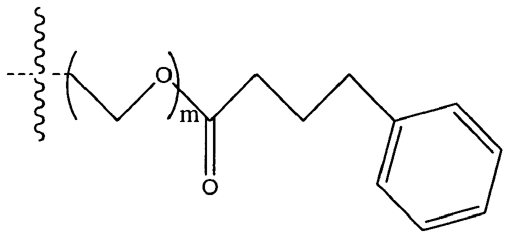 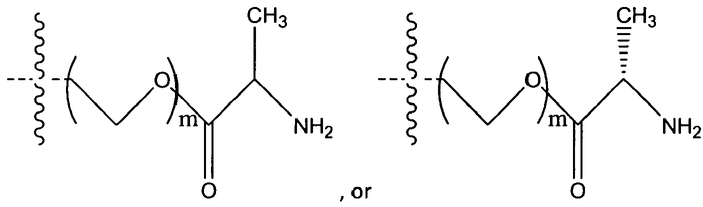 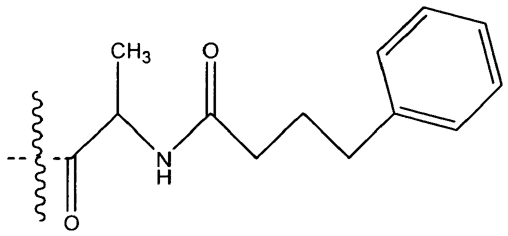 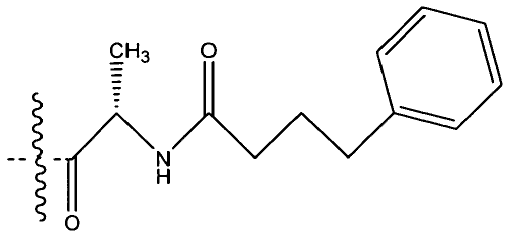 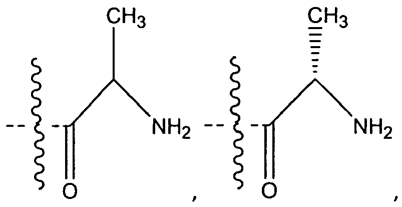 or

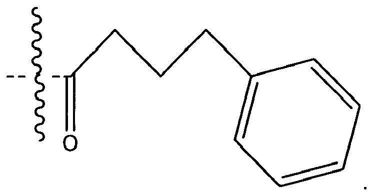

10. Phenylbutyl-Derivative according to any one of claims 1 to 6 and 9 being a compound of Formula II 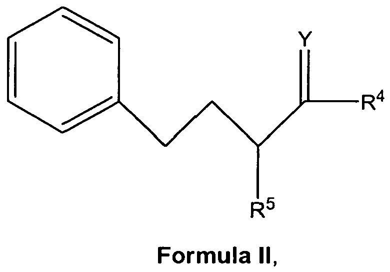 wherein
Y is selected from O or H₂;
R⁵ is selected from H or NH₂; and
R⁴ is selected from a derivative of either a salicylic acid, phenyl butyric acid, ester or ether or a radical of a D-amino acid, being optionally connected to the core by polyethyleneglycol, one or more amino acids or mixtures thereof;
optionally in form of one of its stereoisomers, enantiomers or diastereomers, its racemate or in form of a mixture of at least two of its stereoisomers in any mixing ratio;
in form of the free acid, free base or salt;
preferably, wherein
R⁴ is selected from any of the following structures, which may optionally be substituted by one or more of F, Cl, Br, I, NH₂, OH, SH, CH₃, C₂H₅, C₃H₇, C₄H₉, CH₂Cl, CHCl₂, CF₃, CHF₂, CH₂F: 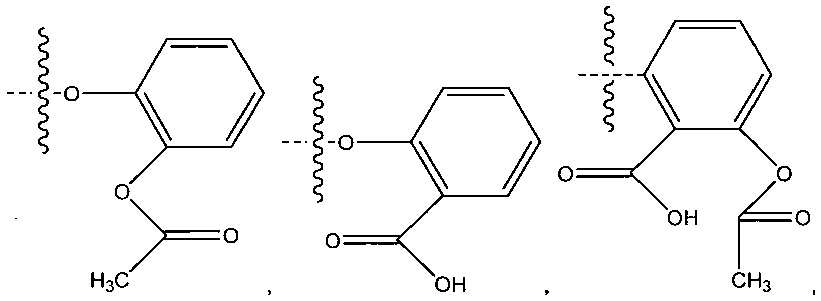 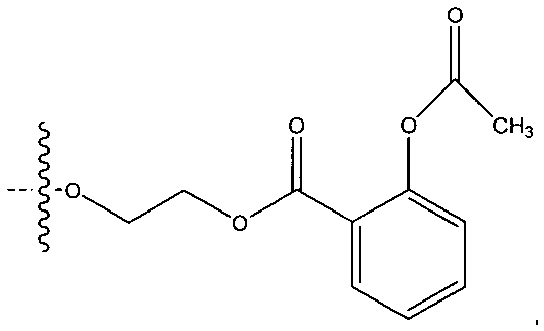 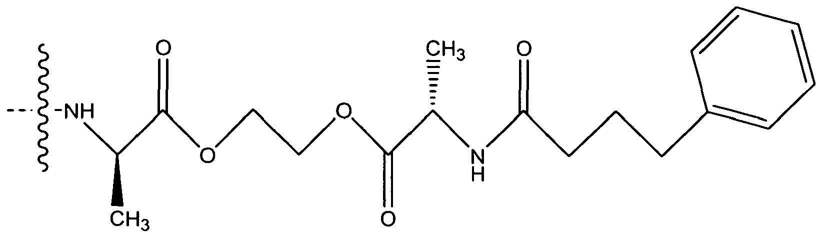 ,a radical of a D-amino acid, or

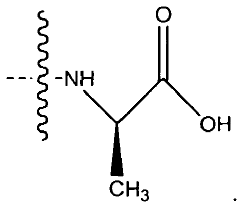

11. Phenylbutyl-Derivative of Formula I according to claims 1 or 10, wherein R⁴ is selected from any one of the following structures: 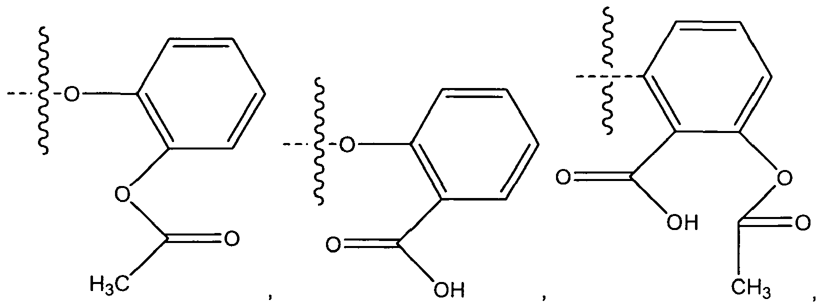 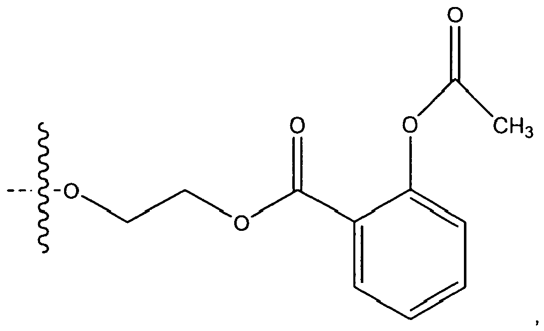 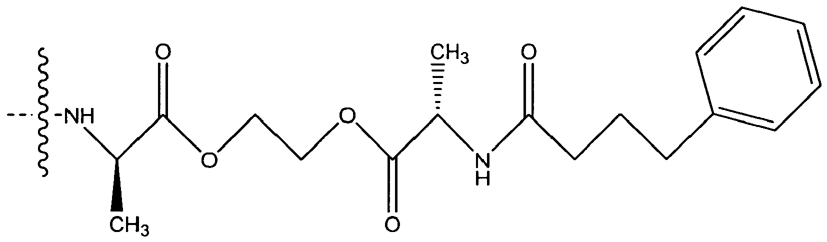 , or

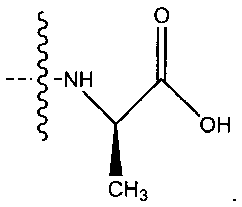

12. Phenylbutyl-Derivative of Formula I according to any of claims 1 to 4, and 10 or 11 selected from 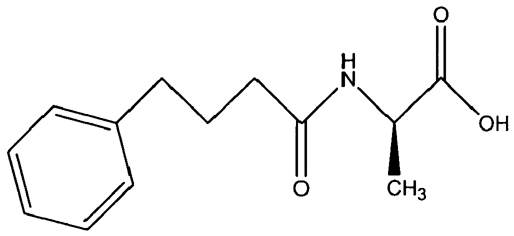 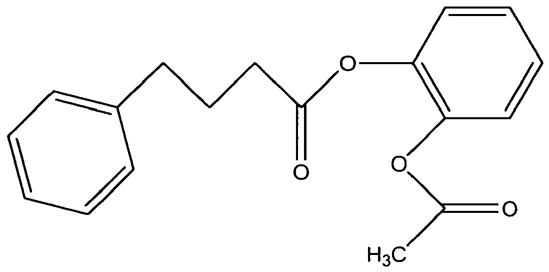 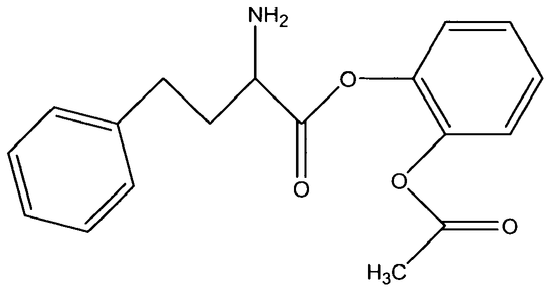 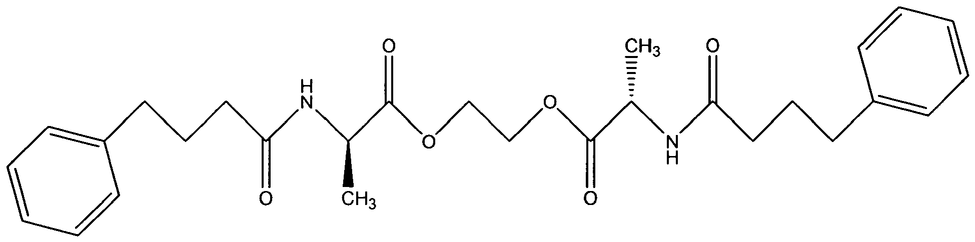 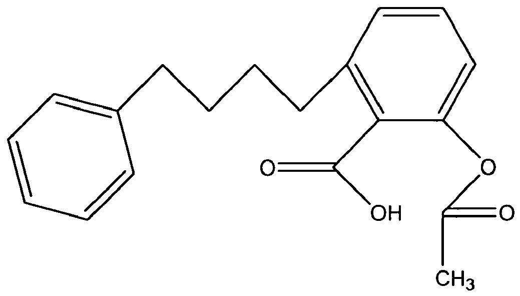 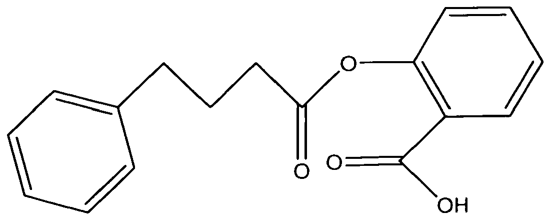 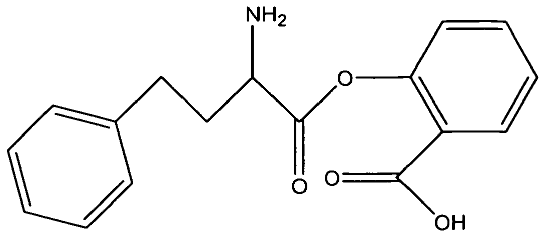 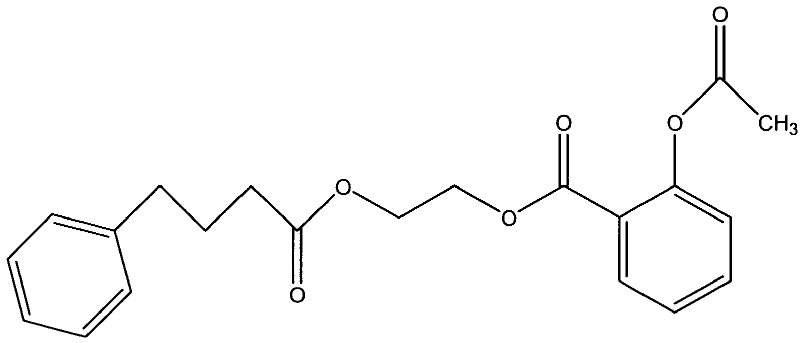 , wherin the compound may optionally be substituted by one or more of F, Cl, Br, I, NH₂, OH, SH, CH₃, C₂H₅, C₃H₇, C₄H₉, CH₂Cl, CHCl₂, CF₃, CHF₂, CH₂F, preferably is unsubstituted;
optionally in form of one of its stereoisomers, enantiomers or diastereomers, its racemate or in form of a mixture of at least two of its stereoisomers in any mixing ratio;
in form of the free acid, free base or salt.

13. Process for producing a Phenylbutyl-Derivative of Formula I according to any of claims 1 to 12, wherein in one part of the process a compound of Formula III 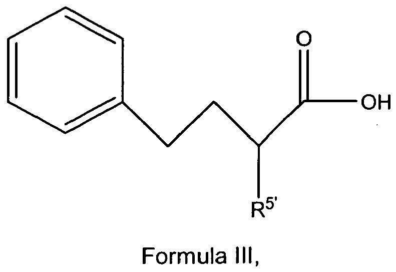 wherein R^{5'} is either hydrogen or 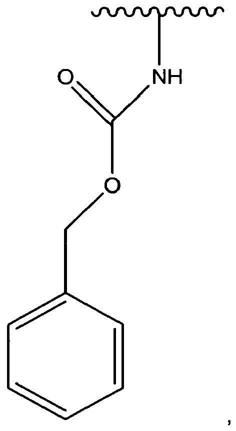 is reacted with a compound of either - if n is # 0 - Formula IV 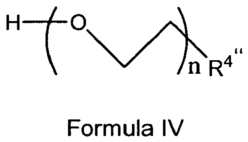 or - if n is 0 - R⁴' with R⁴' being selected from a salicylic acid derivative, radical of an amino acid or of 4-phenyl butyric acid either as free acid, or base or salt thereof,
wherein R^{4"} and R⁴' optionally comprises one or more covalently bound additional amino acids and/or optionally one or more protecting groups PG and/or optionally one or more leaving groups LG like a halogen.

14. Pharmaceutical formulation comprising at least one Phenylbutyl-Derivative of Formula I according to any of claims 1 to 12 and optionally at least one physiologically acceptable excipient.

15. Phenylbutyl-Derivative of Formula I according to any of claims 1 to 12 for use in the treatment of cancer, mucositis, amyotropic lateral sclerosis (ALS), skin/mucosal itching, degenerative diseases of the eye, neurodegenerative disorders, Huntington's disease, eating disorders, obesity, drug induced weight gain, pruritis, alcoholism, promyelocytic leukemia, especially acute promyelocytic leukemia, cardiac injury, especially adriamycin induced cardiac injury, epithelial damage, connective tissue damage, desmosis, Parkinson Disease, myelodysplastic syndrome, urea cycle disorder, fibrotic lung diseases, hepatic encephalopathies, thioredoxin mediated diseases, human papilloma virus (HPV) infections, autoimmune diseases, thalassemia, genetic disorders; or in the regulation of HDAC6 activity, the modulation of stem cells, the prevention of grey hair, the promotion of neuronal growth, the prevention of bone and joint diseases, or as adjuvant in cancer therapy.
